# EUROPEAN PATENT APPLICATION

(11) **EP 3 015 862 A1**
(43) Date of publication of application: **04.05.2016**
(21) Application number: 14190538.0
(22) Date of filing: 27.10.2014
(51) Int. Cl.: G01N 33/542

(54) **Method for the Detection of Antigen Presentation**

(71) Applicant: Deutsches Krebsforschungszentrum Stiftung des Öffentlichen Rechts, 69120 Heidelberg (DE); Ruprecht-Karls-Universität Heidelberg, 69117 Heidelberg (DE)
(72) Inventor: Platten, Michael, 69115 Heidelberg (DE); Schumacher, Theresa, 69115 Heidelberg (DE); Bunse, Lukas, 69115 Heidelberg (DE); Wick, Wolfgang, 69120 Heidelberg (DE); Sahm, Felix, 69115 Heidelberg (DE)
(74) Representative: Krauss, Jan

(57) **Abstract**

The present invention pertains to a method for detecting antigen presentation via antigen presenting molecules such as major histocompatibility complex (MHC) class I or II. The invention deploys a first binding agent specific for the antigen epitope and a second binding agent specific for the antigen presenting molecule. The binding agents of the invention are coupled to proximity probes which upon antigen presentation elicit a detectable signal. The method of the invention allows detecting antigen presentation via MHC *in-vitro* and in a tissue sample *in-situ.* Thus the method of the present invention finds application as a new diagnostic tool, for example in the diagnosis of various diseases such as infectious diseases, immunological disorders, in particular autoimmune diseases, and proliferative disorders such as cancer.

SEQUENCE LISTING

<110>  Deutsches Krebsforschungszentrum Heidelberg
       Ruprecht-Karls-Universität Heidelberg

<120>  Method for the Detection of Antigen Presentation

<130>  D31328EP

<160>  30

<170>  PatentIn version 3.5

<210>  1
<211>  29
<212>  PRT
<213>  Homo sapiens

<400>  1

Pro Arg Leu Val Ser Gly Trp Val Lys Pro Ile Ile Ile Gly Arg His
1               5                   10                  15

Ala Tyr Gly Asp Gln Tyr Arg Ala Thr Asp Phe Val Val
            20                  25

<210>  2
<211>  29
<212>  PRT
<213>  Homo sapiens

<400>  2

Pro Arg Leu Val Ser Gly Trp Val Lys Pro Ile Ile Ile Gly His His
1               5                   10                  15

Ala Tyr Gly Asp Gln Tyr Arg Ala Thr Asp Phe Val Val
            20                  25

<210>  3
<211>  15
<212>  PRT
<213>  Homo sapiens

<400>  3

Pro Arg Leu Val Ser Gly Trp Val Lys Pro Ile Ile Ile Gly Arg
1               5                   10                  15

<210>  4
<211>  15
<212>  PRT
<213>  Homo sapiens

<400>  4

Leu Val Ser Gly Trp Val Lys Pro Ile Ile Ile Gly Arg His Ala
1               5                   10                  15

<210>  5
<211>  15
<212>  PRT
<213>  Homo sapiens

<400>  5

Ser Gly Trp Val Lys Pro Ile Ile Ile Gly Arg His Ala Tyr Gly
1               5                   10                  15


<210>  6
<211>  15
<212>  PRT
<213>  Homo sapiens

<400>  6

Trp Val Lys Pro Ile Ile Ile Gly Arg His Ala Tyr Gly Asp Gln
1               5                   10                  15


<210>  7
<211>  15
<212>  PRT
<213>  Homo sapiens

<400>  7

Lys Pro Ile Ile Ile Gly Arg His Ala Tyr Gly Asp Gln Tyr Arg
1               5                   10                  15


<210>  8
<211>  15
<212>  PRT
<213>  Homo sapiens

<400>  8

Ile Ile Ile Gly Arg His Ala Tyr Gly Asp Gln Tyr Arg Ala Thr
1               5                   10                  15


<210>  9
<211>  15
<212>  PRT
<213>  Homo sapiens

<400>  9

Ile Gly Arg His Ala Tyr Gly Asp Gln Tyr Arg Ala Thr Asp Phe
1               5                   10                  15


<210>  10
<211>  15
<212>  PRT
<213>  Homo sapiens

<400>  10

Arg His Ala Tyr Gly Asp Gln Tyr Arg Ala Thr Asp Phe Val Val
1               5                   10                  15


<210>  11
<211>  15
<212>  PRT
<213>  Homo sapiens

<400>  11

Pro Arg Leu Val Ser Gly Trp Val Lys Pro Ile Ile Ile Gly His
1               5                   10                  15


<210>  12
<211>  15
<212>  PRT
<213>  Homo sapiens

<400>  12

Leu Val Ser Gly Trp Val Lys Pro Ile Ile Ile Gly His His Ala
1               5                   10                  15


<210>  13
<211>  15
<212>  PRT
<213>  Homo sapiens

<400>  13

Ser Gly Trp Val Lys Pro Ile Ile Ile Gly His His Ala Tyr Gly
1               5                   10                  15


<210>  14
<211>  15
<212>  PRT
<213>  Homo sapiens

<400>  14

Trp Val Lys Pro Ile Ile Ile Gly His His Ala Tyr Gly Asp Gln
1               5                   10                  15


<210>  15
<211>  15
<212>  PRT
<213>  Homo sapiens

<400>  15

Lys Pro Ile Ile Ile Gly His His Ala Tyr Gly Asp Gln Tyr Arg
1               5                   10                  15


<210>  16
<211>  15
<212>  PRT

<213>  Homo sapiens

<400>  16

Ile Ile Ile Gly His His Ala Tyr Gly Asp Gln Tyr Arg Ala Thr
1               5                   10                  15


<210>  17
<211>  15
<212>  PRT
<213>  Homo sapiens

<400>  17

Ile Gly His His Ala Tyr Gly Asp Gln Tyr Arg Ala Thr Asp Phe
1               5                   10                  15


<210>  18
<211>  15
<212>  PRT
<213>  Homo sapiens

<400>  18

His His Ala Tyr Gly Asp Gln Tyr Arg Ala Thr Asp Phe Val Val
1               5                   10                  15


<210>  19
<211>  20
<212>  PRT
<213>  Homo sapiens

<400>  19

Gly Trp Val Lys Pro Ile Ile Ile Gly His His Ala Tyr Gly Asp Gln
1               5                   10                  15


Tyr Arg Ala Thr
            20


<210>  20
<211>  20
<212>  PRT
<213>  Homo sapiens

<400>  20

Gly Trp Val Lys Pro Ile Ile Ile Gly His His Ala Tyr Gly Asp Gln
1               5                   10                  15


Tyr Arg Ala Thr
            20


<210>  21
<211>  21
<212>  PRT

<213>  Homo sapiens

<400>  21

Met Glu Val Gly Trp Tyr Arg Ser Pro Phe Ser Arg Val Val His Leu
1               5                   10                  15

Tyr Arg Asn Gly Lys
            20

<210>  22
<211>  19
<212>  RNA
<213>  Homo sapiens

<400>  22
cugaauggau gcugcagau                                                    19

<210>  23
<211>  19
<212>  RNA
<213>  Homo sapiens

<400>  23
ccggcaacau acugacuau                                                    19

<210>  24
<211>  19
<212>  RNA
<213>  Homo sapiens

<400>  24
cgccaugccu uucgcgaca                                                    19

<210>  25
<211>  19
<212>  RNA
<213>  Homo sapiens

<400>  25
gcuggaggag gacggcuua                                                    19

<210>  26
<211>  19
<212>  RNA
<213>  Homo sapiens

<400>  26
ugacaaagcg cuccaacua                                                    19

<210>  27
<211>  19
<212>  RNA
<213>  Homo sapiens

<400>  27

ugaccaauca ggcgaguuu                                                    19

<210>  28
<211>  19
<212>  RNA
<213>  Homo sapiens

<400>  28
ggaaucaugg gcuaucaaa                                                    19

<210>  29
<211>  19
<212>  RNA
<213>  Homo sapiens

<400>  29
caacugagga cguuuacga                                                    19

<210>  30
<211>  13
<212>  PRT
<213>  Homo sapiens

<400>  30

Cys Lys Pro Ile Ile Ile Gly His His Ala Tyr Gly Asp
1               5                   10

## Description

### FIELD OF THE INVENTION

The present invention pertains to a method for detecting antigen presentation via antigen presenting molecules such as major histocompatibility complex (MHC) class I or II. The invention deploys a first binding agent specific for the antigen epitope and a second binding agent specific for the antigen presenting molecule. The binding agents of the invention are coupled to proximity probes which upon antigen presentation elicit a detectable signal. The method of the invention allows detecting antigen presentation via MHC *in-vitro* and in a tissue sample *in*-*situ*. Thus the method of the present invention finds application as a new diagnostic tool, for example in the diagnosis of various diseases such as infectious diseases, immunological disorders, in particular autoimmune diseases, and proliferative disorders such as cancer.

### DESCRIPTION

MHC molecules are classified as either class I or class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs. MHC class II molecules are a family of molecules normally found only on antigen-presenting cells such as dendritic cells, mononuclear phagocytes, some endothelial cells, thymic epithelial cells, and B cells. The antigens presented by class II peptides are derived from extracellular proteins (not cytosolic as in class I); hence, the MHC class II-dependent pathway of antigen presentation is called the endocytic or exogenous pathway. Loading of a MHC class II molecule occurs by phagocytosis; extracellular proteins are endocytosed, digested in lysosomes, and the resulting epitopic peptide fragments are loaded onto MHC class II molecules prior to their migration to the cell surface.

Peptide-MHC binding is generally related to immune activity and/or inactivity, and thereby has implications in a wide range of conditions and diseases, including but not limited to inflammation, allergy, autoimmune diseases, various types of cancers, and infection (viral or bacterial). Patients with diseases associated with immunosuppression, such as cancer, may benefit from strategies to remove immunosuppression and/or enhance tumor- specific immune response. On the contrary, patients with diseases associated with heightened immune activity, such as inflammation, autoimmunity, allergy, and asthma, may benefit from strategies to down-regulate immune responses. Therefore, it is detrimental to monitor whether a peptide antigens suspected to elicit an immune reaction via MHC binding are indeed *in-situ* or *in-vivo* presented via MHC, and thus are immunological active and have the potential to activate the immune system.

Active cancer immunotherapy exploits the patient's immune system to induce cellular immune responses against the tumor, for instance by antigen-specific vaccination. Promising strategies in melanoma, renal cell carcinoma, non-small cell lung carcinoma, glioma and other tumors include DC vaccination using autologous tumor lysate or specific proteins to load DCs *ex vivo,* peptide vaccination, or adoptive transfer of tumor-specific T cells. The use of tumor-associated or tumor-specific antigens (TAA) as targets is considered superior to whole tumor proteome in terms of effective induction of immune responses to overcome tolerance and to preserve integrity of healthy tissue. Vaccination against specific tumor antigens has proven to be a promising tool in therapy for various tumour entities, including gliomas.

For instance, a peptide vaccine against tumor-specific neo-antigens such as the EGFR variant III (EGFRvIII), a constitutively active form that is expressed in 30% of primary gliomas and other tumor entities, is currently tested in phase III clinical trials as an adjunct to radiochemotherapy in patients with newly diagnosed glioblastoma. Classic tumor-associated antigens such as the cancer-testis antigen New York Esophageal 1 (NY-ESO-1), however, are intracellular proteins requiring proteasomal processing and presentation on MHC molecules to induce an antigen-specific T cell response.

To assess MHC binding of a putative or identified epitopic peptide, *in silico* binding algorithms such as NetMHC or SYFPEITHI have been established. These analyses may guide subsequent in vitro class I and class II binding assays, and cellular binding studies, e.g. T2 binding assay. Novel epitopes presented on MHC can be identified by HLA-ligandome analyses, which elute peptides from MHC-peptide complexes from an in vitro system or tumor tissue. These analyses have been successfully applied to develop multi-peptide vaccines, which are currently in clinical trials in patients with various cancers (e.g. IMA 950, NCT01920191).

All of these methods are limited in that they have been mostly developed for MHC class I binding studies and are HLA-type restricted. Binding algorithms at present have limited reliability for MHC class II, and class II peptide complexes are rather instable and therefore difficult to analyse. Some of the tests are very expensive, time-consuming or require freshly isolated tumor tissue. Moreover, they can rarely provide information about the processing and the cellular context in which MHC class II and peptide interaction takes place in situ.

In view of the above described drawbacks in the background art it is to date an unsolved problem that the presentation of antigenic epitopes through binding to MHC cannot be easily monitored in a cellular context. Validation of whether a given epitope is actually presented and possess the ability to mediate immune reactions remains difficult. Thus, the art is in need of improved approaches to detect, preferably directly on a cell *in-sitat,* the binding of antigenic epitopes to their antigen presenting molecules such as the MHC complexes.

The above problem is solved in a first aspect by a method for detecting antigen presentation of an epitope by an antigen presentation molecule, comprising
(a) Providing a first binding agent and a second binding agent, wherein the first binding agent is capable of specifically binding the epitope, and the second binding agent is capable of specifically binding the antigen presentation molecule; wherein the first and the second binding agent are characterized in that spatial proximity of the first binding agent and the second binding agent induces a detectable signal,
(b) Providing an antigen presentation molecule, wherein the antigen presentation molecule is suspected of presenting the antigenic epitope,
(c) Providing the antigenic epitope,
(d) Bringing into contact the antigenic epitope, the antigen presentation molecule, the first binding agent and the second binding agent,
wherein the presence of a detectable signal is indicative for the antigen presentation of the epitope by the antigen presentation molecule.

The term "binding agent" as used herein refers to any molecule capable of specifically binding to a target molecule, preferably the target molecule is the epitope or the antigen presentation molecule of the invention. A binding agent can be selected from an aptamer, an antibody, a receptor molecule, a ligand or a molecular imprinted polymer. Furthermore the term "binding agent" comprises all fragments, multimers or derivatives of the aforementioned agents insofar they maintain the ability to specifically bind their target. The terms "first binding agent", "second binding agent", "third binding agent" or "fourth binding agent" may be selected from any of the aforementioned molecules, independent of each other. Most preferably however, the first to the fourth binding agent in the context of the herein disclosed invention have non-overlapping specificities. Thus, each of the binding agents of the invention has a specific target it binds to, and that specific target does not bind any of the other binding agents of the invention.

The term "capable of binding" as used herein shall mean that the binding agent is under certain conditions able to specifically bind its target. Depending on the kind of binding agent used, these conditions may vary.

As used herein, the terms "specific binding," "selective binding," "selectively binds," and "specifically binding," and the like, when used in context of an antibody as binding agent, refer to binding of the antibody to an epitope on a predetermined antigen. Typically, the antibody binds with an equilibrium dissociation constant (K_{D}) of approximately less than 10⁻⁷ M, such as approximately less than 10⁻⁸ M, 10⁻⁹ M or 10⁻¹⁰ M or even lower when determined by surface plasmon resonance (SPR) technology in a BIACORE 2000 instrument using recombinant antigen/epitope as the analyte and the antibody as the ligand and binds to the predetermined antigen with an affinity that is at least two-fold greater than its affinity for binding to a non-specific antigen (e.g. , BSA, casein) other than the predetermined antigen or a closely-related antigen.

The term "spatial proximity" in context of the invention shall refer to a spatial (3-dimensional) close distance between two binding molecules of the invention. The basic idea of the invention is that epitope presentation via antigen presentation molecules requires a direct binding of the epitope and the antigen presenting molecule. Therefore, if the epitope is presented by the antigen presentation molecule, both are in close spatial proximity to each other, preferably are in direct contact. In this case, the first and the second binding agent of the invention, if bound to their targets, are as well in close spatial proximity. Then, "spatial proximity" of two binding agents of the invention induces a detectable signal according to the invention, in particular if the two targets of the two binding agents of the invention are together in one complex, or at least spatially very close. In certain embodiments of the invention secondary binding agents are used which specifically bind the first and the second binding agent of the invention. In this embodiment, spatial proximity of the first and the second binding agent of the invention causes spatial proximity of the third and fourth binding agent of the invention, if they are bound to their targets. Preferably the two targets of the binding agents of the invention should be in a distance of at least 200nm or less, preferably 100nm or less, more preferably 80nm or less, even more preferably 50 nm or less, most preferably 30-40nm or less. Spatial proximity in preferred embodiments refers to the distance of the binding sites on the targets of the binding agents of the invention.

Yet in a further embodiment of the invention the spatial proximity of the first binding agent and the second binding agent induces directly or indirectly a detectable signal. In this respect the term "directly" shall mean that the first and the second binding agent comprise means that produce a detectable signal upon spatial proximity of the first and second binding agent. The term "indirectly" in this context refers to the first and second binding agent that upon the performance of further method steps finally yield into a detectable signal - under the proviso that the signal is dependent on spatial proximity of the first and second binding agent.

In a preferred embodiment of this aspect of the invention the first binding agent comprises a first proximity probe and the second binding agent comprises a second proximity probe, wherein spatial proximity of the first proximity probe and the second proximity probe induces the detectable signal. In this aspect the "proximity probe" according to the present invention is attached to the binding agent, for example covalently. In this case the "proximity probes" of the invention are sensitive to spatial proximity as defined above and when brought into spatial proximity react such that a detectable signal can be produced. The proximity probes of the invention can be selected from any means known in the art that are sensitive to spatial proximity.

An alternative embodiment of the invention then pertains to the above method further comprising: providing a third binding agent capable of specifically binding to the first binding agent; providing a fourth binding agent capable of specifically binding to the second binding agent; and wherein step (d) comprises bringing into contact the antigenic epitope, the antigen presentation molecule, the first binding agent, the second binding agent, the third binding agent, and the fourth binding agent. Using secondary binding agents may be preferably due to signal amplification.

In context of the alternative embodiment of the use of the third and fourth binding agent, it may be preferred that the third binding agent comprises a first proximity probe and the fourth binding agent comprises a second proximity probe, wherein spatial proximity of the first proximity probe and second proximity probe induces the detectable signal. In this event no proximity probes are attached to the first and second binding agent.

The "binding agents" of the invention may be selected from any molecule that allows a specific biding to a target. Preferably the binding agents of the invention are peptide or nucleic acid aptamers, antibodies or antigen binding fragments of antibodies. Most preferably the binding agents of the invention are antibodies or derivatives or fragments thereof. Antibody derivatives or fragments that still maintain antibody specificity are well known to those skilled in the art. Antibodies of the invention are preferably monoclonal or polyclonal antibodies, preferably monoclonal antibodies, or their derivatives or fragments.

If the above method is used in the embodiment requiring a third and fourth binding agent (antibody), then it can be preferred that the first and second binding agent (antibody) are raised in different species. For example the first and second binding agent of the invention can be selected from an antibody of a mouse, goat, rabbit, rat, guinea pig, monkey, dog, cat, and human, or any other animal capable of producing antibodies upon antigen immunization. This embodiment allows that the third and fourth antibodies are antibodies specific for the respective antibody species of the first and second binding agent. For example if the first binding agent is a rat antibody, and the second binding agent is a rabbit antibody, the third binding agent is exemplary a goat anti-rat antibody, and the fourth binding agent is exemplary a goat anti-rabbit antibody. Theoretically all permutations of antibody species are possible in context of the present invention.

In the context of the present invention the term "epitope" as used herein, may include, but is not limited to, a nucleotide, a carbohydrate, a protein or peptide, a lipid, a capsid protein, a polysaccharide, a lipopolysaccharide, a glycolipid, a glycoprotein, and/or or at least a part of a cell. As used herein, the term "epitope" may be used interchangeably with antigen, paratope binding site, antigenic determinant, and/or determinant. As used herein, the term determinant can include an influencing or determining element or factor, unless context indicates otherwise. In one aspect the term "epitope" includes, but is not limited to, a peptide binding site. In this event the peptide epitope of the invention includes peptides comprising an amino acid sequence that has a maximum length of 100 amino acids, preferably 90, 80, 70, 60, 50, 40, 30, 20, 19, 18, 17, 16, 15, 14, 13, 12, 11, 10, 9 amino acids. Preferably the peptides have a minimum length of 7 to 14, and preferably comprise/consist of 8, 9, 10, 11, 12, 13, or 14 amino acids.

In preferred embodiments of the invention the epitope of the invention is a peptide epitope, preferably a MHC class I or MHC class II, most preferably a MHC class II peptide epitope. For MHC Class I epitopes, it is preferred that the peptides have between 5 to 20 amino acids (e.g., between 7 and 15 amino acids, or between 8 and 11 amino acids (e.g., 8, 9, 10, or 11 amino acids in length)). MHC class II peptides in accordance with the present invention preferably have an overall length of between 8 and 100, preferably between 8 and 30, and most preferred between 8 and 16, namely 8, 9, 10, 11, 12, 13, 14, 15 or 16 amino acids.

The epitope of the invention is preferably an epitope derived of a disease associated antigen, preferably an epitope associated with an immunological disorder, such as an autoimmune disorder, or a tumor associated antigen (TAA), or an epitope derived from a TAA, preferably wherein the TAA is selected from the group of cancer mutated antigens, cancer germ line expressed antigens, cancer viral antigens or cancer overexpression antigens. Most preferred examples of such epitiopes are the epitopes used in exemplary description of the herein disclosed invention (see below under "Examples"). Therefore the epitope can be derived from IDH1 and comprises the IDH1R132H mutation, or wherein the epitope is derived from NY-ESO. Although these antigenic epitopes are preferred, they should not be understood as a limitation of the methods of the present invention.

In this regard, it is in certain embodiments preferred that the antigen presentation molecule is a MHC class I or MHC class II molecule, or any protein complex comprising a MHC I or MHC II molecule, preferably a MHC class II molecule. The human variants of MHC are denoted human leucocyte antigen (HLA) complex. The antigen presentation molecule in preferred embodiments of the invention is selected from a MHC class I or II molecule. However, it is also possible to use any protein that is in the same complex with MHC or directly associated with the antigen presentation function of the complex, in particular if this protein is in close spatial proximity with the presented epitope during antigen presentation.

In this preferred embodiment of the invention the first binding agent is an antibody specifically capable of binding a candidate peptide epitope, and the second binding agent is an antibody specifically binding an MHC complex. If the epitope is presented via MHC class I, said MHC antibody is specific for MHC class I, and if the epitope is presented via MHC class II, said MHC antibody is specific for MHC class II. Such anti-MHC antibodies are known in the art.

In accordance with the herein described invention a "detectable signal" refers to a change in or appearance of a property in a reporter system which is capable of being perceived or sensed, either by direct observation or instrumentally, and which is a function of the presence of the epitope presentation (epitope/antigen presentation molecule interaction or binding) in a test sample. Some examples of detectable signals are changes in visible or infra-red absorption fluorescence, phosphorescence or chemiluminescence. Other examples of detectable signals may be directed to a change in electrochemical property. Preferred is the emission of light. Most preferred is that the detectable signal is produced via a proximity ligation assay (PLA).

The term "proximity ligation assay" or "PLA" as used herein refers to an assay that involves contacting the analytes - the term "analytes" preferably refers to the epitope and the antigen presentation molecule as defined herein above - with at least two proximity detection agents, wherein a first agent comprises the first binding agent and a first oligonucleotide moiety as first proximity probe and the second probe comprises the second binding agent and a second oligonucleotide moiety as second proximity probe. The oligonucleotide moiety of each agent may be the same or different. In some embodiments, the oligonucleotide moiety of each agent in a set of proximity detection agents comprises a different sequence. In some embodiments, the analytes are contacted with a set of proximity detection agents. In some embodiments, a set of proximity detection agents comprises 2, 3, 4, 5, or more than 5 proximity detection agents. In some embodiments, a set of proximity detection agents is a pair of proximity detection agents, or a "proximity detection agent pair."

In one additional embodiment the PLA involves two proximity detection agents, wherein a first agent comprises the third binding agent and a first oligonucleotide moiety as first proximity probe and the second probe comprises the fourth binding agent and a second oligonucleotide moiety as second proximity probe. Herein the third binding agent is capable of specifically binding the first binding agent as described above, and the fourth binding agent is capable of specifically binding the second binding agent.

In some embodiments, after contacting one or more analyte with at least two proximity detection agents, the oligonucleotide moieties of at least two of the proximity detection agents are capable of interacting with one another. In some embodiments, such interaction may be mediated by one or more additional oligonucleotides. In some embodiments, at least a portion of each of the oligonucleotide moieties of the proximity detection agents hybridizes to another oligonucleotide. For example, in some embodiments, at least one additional oligonucleotide is added (referred to herein as a "splint oligonucleotide"), which mediates the interaction between at least two proximity detection agents by hybridizing to at least a portion of the oligonucleotide moiety of each of the proximity detection agents.

In some embodiments, the oligonucleotide moieties of at least two of the proximity detection agents are capable of undergoing chemical ligation. In some embodiments, the ligatable ends of each of the oligonucleotide moieties are brought together by a splint oligonucleotide that is capable of hybridizing to at least a portion of the oligonucleotide moiety of each proximity detection agent.

Following chemical ligation of the oligonucleotide moieties of at least two proximity detection agents, the ligated oligonucleotide moieties may be detected by any method known in the art. In some such embodiments, the ligated oligonucleotide moieties are referred to as a "target nucleic acid." Exemplary methods of detecting the ligated oligonucleotide moieties (or "target nucleic acid") include, but are not limited to, direct detection, real-time PCR (including, but not limited to, 5'-nuclease real-time PCR), rolling circle amplification, combinations of ligation and PCR, and amplification followed by a detection step (such as a second amplification, direct detection, ligation, etc.). Nonlimiting exemplary methods of detecting nucleic acids are described herein.

Exemplary proximity detection assays are described, e.g., in U.S. Pat. No. 6,511,809 B2; U.S. Patent Publication No. US 2002/0064779; PCT Publication No. WO 2005/123963; U.S. Provisional Application No. 61/362,616, filed Jul. 8, 2010; and Gustafsdottir et al., Clin. Chem. 52: 1152-1160 (2006).

The term "quantitative nucleic acid detection assay" as used herein refers to an assay that is capable of quantitating the amount of a particular nucleic acid sequence in a sample. Nonlimiting exemplary quantitative nucleic acid detection assays are described herein.

As used herein, the term "detector probe" refers to a molecule used in an amplification reaction that facilitates detection of the amplification product. Exemplary amplification reactions include, but are not limited to, quantitative PCR, real-time PCR, and end-point analysis amplification reactions. In some embodiments, such detector probes can be used to monitor the amplification of a target nucleic acid and/or control nucleic acid. In some embodiments, detector probes present in an amplification reaction are suitable for monitoring the amount of amplicon(s) produced as a function of time.

In some embodiments, a detector probe is "sequence-based," meaning that it detects an amplification product in a sequence-specific manner. As a non-limiting example, a sequence-based detector probe may comprise an oligonucleotide that is capable of hybridizing to a specific amplification product. In some embodiments, a detector probe is "sequence-independent," meaning that it detects an amplification product regardless of the sequence of the amplification product.

Detector probes may be "detectably different," which means that they are distinguishable from one another by at least one detection method. Detectably different detector probes include, but are not limited to, detector probes that emit light of different wavelengths, detector probes that absorb light of different wavelengths, detector probes that scatter light of different wavelengths, detector probes that have different fluorescent decay lifetimes, detector probes that have different spectral signatures, detector probes that have different radioactive decay properties, detector probes of different charge, and detector probes of different size. In some embodiments, a detector probe emits a fluorescent signal.

The term "detectable label," as used herein, refers to a moiety that is directly or indirectly detectable. In some embodiments, and as a non-limiting example, a detectable label may be directly detectable, e.g., due to its spectral properties. In some embodiments, and as a non-limiting example, a detectable label may be indirectly detectable, e.g., due to its enzymatic activity, wherein the enzymatic activity produces a directly detectable signal. Such detectable labels include, but are not limited to, radiolabels; pigments, dyes, and other chromogens; spin labels; fluorescent labels (i.e., fluorophores such as coumarins, cyanines, benzofurans, quinolines, quinazolinones, indoles, benzazoles, borapolyazaindacenes, and xanthenes, including fluoresceins, rhodamines, and rhodols); chemiluminescent substances, wherein the detectable signal is generated by chemical modification of substance; metal-containing substances; enzymes, wherein the enzyme activity generates a signal (such as, for example, by forming a detectable product from a substrate; haptens that can bind selectively to another molecule (such as, for example, an antigen that binds to an antibody; or biotin, which binds to avidin and streptavidin). Many detectable labels are known in the art, some of which are described, e.g., in Richard P. Haugland, Molecular Probes Handbook of Fluorescent Probes and Research Products (9th edition, CD-ROM, September 2002), supra. In some embodiments, the detectable label comprises a chromophore, fluorophore, fluorescent protein, phosphorescent dye, tandem dye, particle, hapten, enzyme, or radioisotope. In some embodiments, the fluorophore is a xanthene, coumarin, cyanine, pyrene, oxazine, borapolyazaindacene, or carbopyranine. In some embodiments, the enzyme is horseradish peroxidase, alkaline phosphatase, beta-galactosidase, or beta-lactamase. In some embodiments, the particle is a semiconductor nanocrystal.

"Endpoint polymerase chain reaction" or "endpoint PCR" is a polymerase chain reaction method in which the presence or quantity of nucleic acid target sequence is detected after the PCR reaction is complete, and not while the reaction is ongoing.

"Real-time polymerase chain reaction" or "real-time PCR" is a polymerase chain reaction method in which the presence or quantity of nucleic acid target sequence is detected while the reaction is ongoing. In some embodiments, the signal emitted by one or more detector probes present in a reaction composition is monitored at multiple time points during the PCR as an indicator of synthesis of a primer extension product. In some embodiments, fluorescence emitted at multiple time points during the PCR is monitored as an indicator of synthesis of a primer extension product. In some embodiments, the signal is detected during each cycle of PCR.

A "multiplex amplification reaction" is an amplification reaction in which two or more target nucleic acid sequences and/or control nucleic acid sequences are amplified in the same reaction. A "multiplex polymerase chain reaction" or "multiplex PCR" is a polymerase chain reaction method in which two or more target nucleic acid sequences and/or control nucleic acid sequences are amplified in the same reaction.

A "singleplex amplification reaction" is an amplification reaction in which only one target nucleic acid sequence or control nucleic acid sequence is amplified in the reaction. A "singleplex polymerase chain reaction" or "singleplex PCR" is a polymerase chain reaction method in which only one target nucleic acid sequence or control nucleic acid sequence is amplified in the reaction.

"Threshold cycle" or "CT" is defined as the cycle number at which the observed signal from a quantitative nucleic acid detection assay exceeds a fixed threshold. In some embodiments, the fixed threshold is set as the amount of signal observed in a reaction lacking a target nucleic acid sequence or control nucleic acid sequence. In some embodiments, the fixed threshold is set at a level above the background noise signal. For example, in some embodiments, the fixed threshold is set at a value corresponding to 3 or more times the combination of the root mean squared of the background noise signal and the background noise signal. In some embodiments, the observed signal is from a detector probe. In some embodiments, the observed signal is from a fluorescent label.

The term "solid support" as used herein refers to any solid substance that can be mixed or contacted with a liquid and then separated from the liquid. Separation from the liquid may comprise, in some embodiments, centrifugation, use of a magnet, filtration, settling, pipetting, etc. Nonlimiting exemplary solid supports include microparticles (such as polymer beads, metal particles, magnetic beads, etc.), microtiter plates (such as 96-well plates, 384-well plates, 1536-well plates, etc.), and microarray chips. In some embodiments, a solid support comprises a coating that facilitates binding of, for example, a covalent analyte binding moiety and/or a non-covalent analyte binding moiety and/or an oligonucleotide moiety. In some embodiments, the coating comprises a first member of a binding pair. In some such embodiments, a covalent analyte binding moiety and/or a non-covalent analyte binding moiety and/or an oligonucleotide moiety comprises a second member of the binding pair.

The term "solid support particle," as used herein, refers to solid support microparticles. Nonlimiting exemplary solid support particles include polymer beads, metal particles, glass beads, and magnetic beads.

Some preferred embodiments of the invention include that in step (b) the antigen presentation molecule is provided in a biological cell, preferably on the surface on a biological cell such as an antigen presenting cell selected from a dendritic cell, a B lymphocyte or a tumor cell. Additional cells which can be used and that comprise an antigen presentation molecule in accordance with the present invention are microglial cells, endothelial cells, or monocytes, such as mononuclear phagocytes, including classical, non-classical and intermediate monocytes.

As used herein, the term "microglial cell" refers to the macrophage like glial cells found in the central nervous system which release pro-inflammatory substances when activated and includes mononuclear phagocytes and macrophages.

As used herein, the term "mononuclear phagocyte" is an immune cell found in blood and body tissues, including the central nervous system and brain, and include, for example, microglia cells, nionocytes, macrophages, histiocytes, dendritic cells, precursor cells of microglia, precursor cells of monocytes, precursor cells of macrophages, microglia-like cell lines, macrophage-like cell lines, or cell lines.

In some embodiments the cell of the invention comprising the antigen presentation molecule such as MHC, is provided in a cellular suspension, for example in a state of the art cell culture system. Such cells can be floating cells or adhered cells. Further preferred is that the cell of the invention is provided in the context of a tissue sample. Such a tissue sample can be of a tissue affected by a disease treatable or diagnosable by any of the herein disclosed methods or products. The term "tissue sample" (the term "tissue" is used interchangeably with the term "tissue sample") should be understood to include any material composed of one or more cells, either individual or in complex with any matrix or in association with any chemical. The definition shall include any biological or organic material and any cellular subportion, product or by-product thereof. One non limiting example of tissues preferred is a tumor tissue sample. Tissue samples are preferably provided as paraffin embedded tissue samples.

Furthermore, the epitope of the present invention can also be provided in a cellular context (in a biological cell). The cell is preferably the identical cell that also comprises the antigen presentation molecule of the invention. For example the epitope or a precursor thereof of the invention can be endogenously expressed in a cell. Alternatively the cell can ectopically express the epitope, or a precursor thereof, via one or more expression constructs. Preferred cell types are identical to the cell types and tissues mentioned before.

The term "expression construct" in the present context means any double-stranded DNA or double-stranded RNA designed to transcribe an RNA, e.g., a construct that contains at least one promoter operably linked to a downstream gene or coding region of interest (e.g., a cDNA or genomic DNA fragment that encodes a protein or fragment thereof, or any RNA of interest). Transfection or transformation of the expression construct into a recipient cell allows the cell to express RNA or protein encoded by the expression construct. An expression construct may be a genetically engineered plasmid, virus, or an artificial chromosome derived from, for example, a bacteriophage, adenovirus, retrovirus, poxvirus, or herpesvirus, or any other expression vector known to those skilled in the art. An expression construct can be replicated in a living cell, or it can be made synthetically. For purposes of this application, the terms "expression construct", "expression vector", "vector", and "plasmid" are used interchangeably to demonstrate the application of the invention in a general, illustrative sense, and are not intended to limit the invention to a particular type of expression construct.

Further, the term expression construct or vector is intended to also include instances wherein the cell utilized for the assay already endogenously comprises such DNA sequence.

Method step (d) in accordance with the present invention thus comprises providing a biological cell comprising the antigen presentation molecule, or alternatively expressing the antigen presentation molecule in the cell; adding to the cell, or expressing in the cell, the epitope or a precursor thereof; adding to the cell the first and the second binding agent, optionally, the third and fourth binding agent.

In preferred embodiments of the present invention the first and second proximity probe is a nucleic acid, preferably a DNA molecule. For example, the first and the second proximity probe comprise nucleic acid sequences that are complementary to at least two linear nucleic acid templates, and wherein the first and the second proximity probe can be ligated to the two linear nucleic acid templates to form a circular nucleic acid molecule. In this embodiment the "proximity probe" is a oligonucleotide moiety as describe herein above.

The method of the present invention may comprise an additional step of (e) determining whether a detectable signal is present or not. Determining the presence of the detectable signal may comprises a step of nucleic acid ligation, preferably also a PCR amplification step, such as a rolling circle PCR amplification (see above).

Preferred embodiments of the present invention relate to the aforementioned method, which is an *ex vivo, in vitro* or most preferably an *in situ* method.

The afore-mentioned problem of state of the art approaches is furthermore solved by a method for generating a personalized disease therapy plan for treating a subject suffering from a disease. In this aspect the method comprises the steps of:
(a) Providing a biological sample obtained from the subject,
(b) Detecting antigen presentation of at least one known epitope or antigen in the biological sample using the method for detecting antigen presentation of an epitope by an antigen presentation molecule as described herein above, wherein the epitope and the antigen presentation molecule are provided in the biological sample, and
(c) Generating a therapy plan for treating the subject by selecting a vaccine composition comprising vaccine-molecules corresponding to the epitope or antigen as detected in (a).

The problem of the invention is solved in yet a further aspect which pertains to a method for producing a personalized vaccine composition, the method comprising the steps of
(a) Providing a biological sample obtained from the subject,
(b) Detecting antigen presentation of at least one known epitope or antigen in the biological sample using the method for detecting antigen presentation of an epitope by an antigen presentation molecule as described herein above, wherein the epitope and the antigen presentation molecule are provided in the biological sample, and
(c) Producing a personalized vaccine composition by admixing vaccine compounds into a composition which correspond to the epitopes/antigens as detected in (b).

Also comprised by the present invention are vaccine compositions produced with the aforementioned method.

Also, these methods are in preferred embodiments an *ex vivo, in vitro* or most preferably an *in situ* method.

A particular application of the methods of the present invention is in so-called "personalized medicine". The term "personalized medicine" is used herein in its broadest context to refer to the tailoring of pharmaceutical compositions and medicines for particular individuals based on and taking into consideration knowledge of the individual's phenotype and/or genotype. Thus, in selecting a composition or medicine to be administered to any particular individual, use is made of information such as the presentation of disease specific epitopes on a patients tissue affected by the disease. Further information that could be used is the individual's medical history, clinical data and/or the individual's genotype in an attempt to ensure that the composition or medicine is particularly suited to the individual at the time of administration. "Personalized medicine" has the potential to revolutionise the provision of healthcare, however to date little success has been achieved. Embodiments of the present invention provide novel avenues and approaches for the development and delivery of personalized medicine. Using the methods for antigen/epitope presentation on a diseased tissue allows, in particular tumor tissue, allows for a targeted selection of vaccines in order to strengthen the patient's immune system. In a cancer treatment, a patient suffering from a particular cancer can therefore in advance of a treatment be screened for the antigenicity of the diagnosed tumor. Depending on the epitopes found to be presented on the tumor tissue with the methods of the invention, a vaccination treatment plan as well as the design of a personalized vaccine composition comprising specific tumor peptide vaccines can be developed.

In preferred embodiments of the aspect of a personalized medicine is that the disease is selected from an immunological disorder, such as autoimmune diseases, and cancer diseases. Most preferably the method of this aspect finds application in the context of a cancer disease. Then the tissue sample is preferably tumor tissue sample, and the detecting antigen presentation is performed in the tumor tissue sample *in-situ.* Further the epitope or antigen is a tumor associated antigen (TAA), which can be selected depending on the tumor disease from a mutated tumor antigen, germ-line expressed tumor antigen, viral expressed tumor antigen or overexpressed tumor antigen.

In order to generate a treatment plan, and in particular a powerful vaccine composition, it is preferably that not only the presentation of one single epitope or antigen is detected on the tissue sample, but the detection is conducted for at least 2, preferably 3, 4, 5, 6, 7, 8, 9, 10, 11, 12, 13, 14, 15 or more epitopes. In the following a multitude of such epitopes/antigens is referred to as a "panel". By testing a panel of several epitopes, the methods of the invention renders possible to generate a vaccine composition comprising only such vaccines targeting epitopes/antigens that are presented on the tumor of the individual tested. Preferred is the testing of a panel of known TAAs.

The term "vaccine" refers to a composition that can be used to elicit protective immunity in a recipient. It should be noted that to be effective, a vaccine of the invention can elicit immunity in a portion of the immunized population, as some individuals may fail to mount a robust or protective immune response, or, in some cases, any immune response. This inability may stem from the individual's genetic background or because of an immunodeficiency condition (either acquired or congenital) or immunosuppression (e.g., due to treatment with chemotherapy or use of immunosuppressive drugs). Preferably a vaccine according to the invention is a peptide vaccine, which is a vaccine composition comprising one or more peptides or variants thereof.

In the context of the present invention the term "patient", "subject" or "individual" are used interchangeably and preferably refers to a mammal, preferably a human patient. Preferably the mammal or human is an individual suffering from one or more diseases.

Yet a further aspect of the present invention relates to a method for diagnosing, stratifying, monitoring or classifying a subject suffering from a disease, the method comprising the steps of:
(a) Providing a biological sample of the subject suffering from a disease to be diagnosed,
(b) Detecting antigen presentation of at least one known epitope or antigen in the biological sample, the epitope being characteristic for a candidate disease, using the method for detecting antigen presentation of an epitope by an antigen presentation molecule as described herein above, wherein the epitope and the antigen presentation molecule are provided in the biological sample,
wherein a diagnosis is provided based on the presence or absence of the presentation of the epitope/antigen in said cellular sample or tissue sample.

For the purpose of the present invention, the term "biological sample" is intended to mean any sample taken from a subject and liable to contain a biological material (biological cells in particular) as defined hereinafter. This biological sample may in particular be a blood, serum, saliva, tissue, tumor or bone marrow sample or a sample of circulating cells from the patient. This biological sample is obtained by any means for taking a sample known to those skilled in the art.

For the purpose of the present invention, the term "biological material" is intended to mean any material which renders possible the detection of the presentation of epitopes or antigens by antigen presentation molecules, such as MHC proteins, in accordance with the invention. Thus, the biological sample of the invention in particular is a sample containing cells which express either MHC class I or MHC class II proteins.

Via detection of antigen/epitope presentation in accordance with the herein described invention, it is rendered possible to monitor the presentation of diseases associated in a cellular sample obtained from a subject to be diagnosed. For example the diagnostic aspect can find application in the detection of infectious diseases, immunological disorders or tumor disease (see also below).

The methods and compositions of the invention may be used for any disease where a, for use in the treatment of cancer.

Diseases that can be subject to the various methods and compositions of the invention are preferably any disease or condition that is characterized by the differential presentation of antigenic epitopes on the cellular surface on the diseases tissue or cell.

A disease may be preferably selected from the group of infectious diseases. The term "infectious disease" includes those pathologic conditions that arise from bacterial, viral or fungus organisms that invade and disrupt the normal function of the mammalian body. Antigenic epitopes characteristic for a given pathogen are presented on antigen presenting cells, and this presentation can be detected with the methods of the present invention.

Another preferred embodiment relates to the use of the herein described methods and compositions of the invention where the disease is an autoimmune disease. As used herein the term 'autoimmune disease(s)' refers to the group of diseases including obstructive airways disease (including conditions such as COPD (Chronic obstructive pulmonary disease)), asthma (e.g intrinsic asthma, extrinsic asthma, dust asthma, infantily asthma) particularly chronic or inveterate asthma (for example late asthma and airway hyperreponsiveness), bronchitis, including bronchial asthma, systemic lupus erythematosus (SLE), multiple sclerosis, type I diabetes mellitus and complications associated therewith, atopic eczema (atopic dermatitis), contact dermatitis and further eczematous dermatitis, inflammatory bowel disease (e.g. Crohn's disease and ulcerative colitis), atherosclerosis and amyotrophic lateral sclerosis. Particularly the term refers to COPD, asthma, psoriasis, systemic lupus erythematosis, type I diabetes mellitus, vasculitis and inflammatory bowel disease.

In particular preferred is the use of the aforementioned methods and compositions for cancer treatment, prevention or diagnosis. Preferably the cancer is selected from the group comprising cervical cancer, lung cancer, pancreas cancer, non-small cell lung cancer, liver cancer, colon carcinoma, cancer of a bone, skin cancer, cancer of the head and neck, cutaneous or intraocular melanoma, uterine carcinoma, ovarian cancer, rectal cancer, stomach cancer, cancer of the anal region, breast cancer, oviduct cancer, endometrial carcinoma, vaginal cancer, vulva cancer, Hodgkin's disease, esophageal cancer, small intestine tumor, endocrine gland's cancer, thyroid cancer, parathyroid cancer, epinephros cancer, soft tissue sarcomas, urethrophyma, penis cancer, prostatic carcinoma, bladder cancer, kidney and ureter cancer, preferably, cervical cancer, lung cancer, liver cancer, colon carcinoma, skin cancer, stomach cancer, prostatic carcinoma, brain cancer, such as glioma, astrocytoma, or kidney cancer.

One additional aspect of the invention then pertains to a use of a proximity ligation assay (PLA) in a method for detecting antigen presentation of an epitope by an antigen presentation molecule as described herein above.

Yet one further aspect pertains to a use of a PLA kit in a method for detecting antigen presentation of an epitope by an antigen presentation molecule as described herein above, wherein the kit comprises at least the first binding agent and the second binding agent, optionally the kit further comprises the second binding agent and the fourth binding agent.

Also provided is a diagnostic kit adapted to be for use in the performance of a method for detecting antigen presentation of an epitope by an antigen presentation molecule as described herein above, the diagnostic kit comprising at least the first binding agent, the second binding agent, optionally the third and fourth binding agent. The diagnostic kit may further comprise materials useful for the performance of a PLA assay, such as a PLA positive probe, a PLA negative probe.

The problem of the present invention is furthermore solved by a proteinaceous complex, the complex comprising an epitope, and antigen presentation molecule, the first binding agent, and the second binding agent. In this aspect, the aforesaid for the epitope, the antigen presentation molecule and the binding agents similarly apply for this aspect.

The present invention allows an improvement of vaccine-based treatments of various diseases. The vaccines in accordance with the invention can be formulated in pharmaceutical compositions. These compositions may comprise, in addition to one of the above substances, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal or patch routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Whether it is a polypeptide, peptide, or nucleic acid molecule, other pharmaceutically useful compound according to the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, e.g. in a viral vector (a variant of the VDEPT technique-see below). The vector could be targeted to the specific cells to be treated, or it could contain regulatory elements, which are switched on more or less selectively by the target cells.

Alternatively, the agent could be administered in a precursor form, for conversion to the active form by an activating agent produced in, or targeted to, the cells to be treated (prodrug). This type of approach is sometimes known as ADEPT or VDEPT; the former involving targeting the activating agent to the cells by conjugation to a cell-specific antibody, while the latter involves producing the activating agent, e.g. a vaccine or fusion protein, in a vector by expression from encoding DNA in a viral vector.

In a specific embodiment of the present invention, nucleic acids are provided which include a sequence that encodes a vaccine, or functional derivatives thereof, and are administered to modulate immune cell activation by way of gene therapy. In more specific embodiments, a nucleic acid or nucleic acids encoding a vaccine or fusion protein, or functional derivatives thereof, are administered by way of gene therapy. Gene therapy refers to therapy that is performed by the administration of a specific nucleic acid to a subject. In this embodiment of the present invention, the nucleic acid produces its encoded peptide(s), which then serve to exert a therapeutic effect by modulating function of the disease or disorder. Any of the methodologies relating to gene therapy available within the art may be used in the practice of the present invention.

In a preferred embodiment, a therapeutic of the invention comprises a nucleic acid that is part of an expression vector expressing any one or more of the vaccines, fusion proteins, or fragments, derivatives or analogs thereof, within a suitable host. In a specific embodiment, such a nucleic acid possesses a promoter that is operably-linked to coding region(s) of a fusion protein. The promoter may be inducible or constitutive, and, optionally, tissue-specific. In another specific embodiment, a nucleic acid molecule is used in which coding sequences (and any other desired sequences) are flanked by regions that promote homologous recombination at a desired site within the genome, thus providing for intra-chromosomal expression of nucleic acids.

Delivery of the therapeutic nucleic acid into a subject (patient) may be either direct (i.e., the patient is directly exposed to the nucleic acid or nucleic acid-containing vector) or indirect (i.e., cells are first transformed with the nucleic acid in vitro, then transplanted into the patient). These two approaches are known, respectively, as *in vivo* or *ex vivo* gene therapy. In a specific embodiment of the present invention, a nucleic acid is directly administered *in vivo,* where it is expressed to produce the encoded product. This may be accomplished by any of numerous methods known in the art including, e.g., constructing the nucleic acid as part of an appropriate nucleic acid expression vector and administering the same in a manner such that it becomes intracellular; directly injecting naked DNA; using microparticle bombardment; coating the nucleic acids with lipids; using associated cell-surface receptors/transfecting agents; encapsulating in liposomes, microparticles, or microcapsules; administering it in linkage to a peptide that is known to enter the nucleus; or by administering it in linkage to a ligand predisposed to receptor-mediated endocytosis.

The vaccines of the present invention also include one or more adjuvant compounds. Adjuvant compounds are useful in that they enhance long term release of the vaccine by functioning as a depot. Long term exposure to the vaccine should increase the length of time the immune system is presented with the antigen for processing as well as the duration of the antibody response. The adjuvant compound also interacts with immune cells, e.g., by stimulating or modulating immune cells. Further, the adjuvant compound enhances macrophage phagocytosis after binding the vaccine as a particulate (a carrier/vehicle function).

The present invention will now be further described in the following examples with reference to the accompanying figures and sequences, nevertheless, without being limited thereto. For the purposes of the present invention, all references as cited herein are incorporated by reference in their entireties. In the Figures:

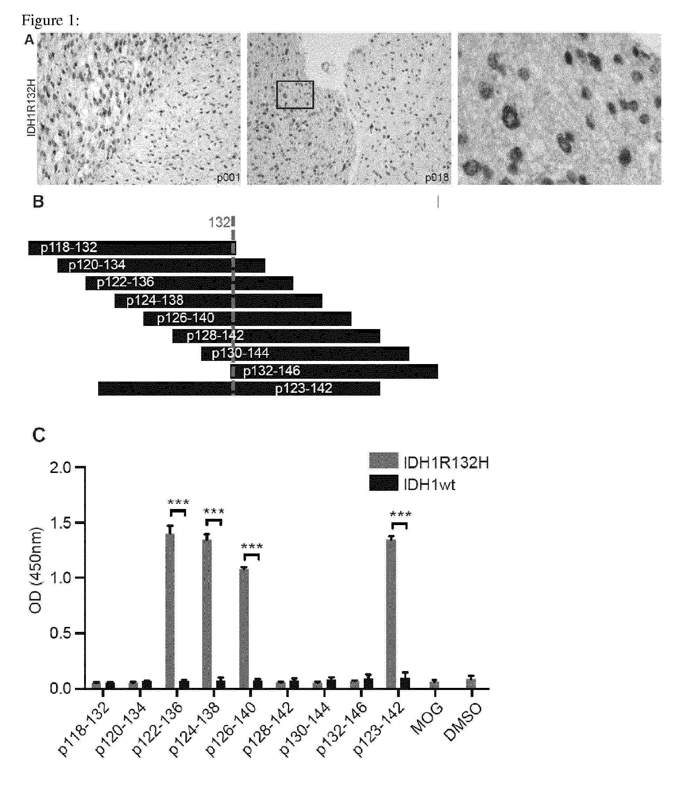

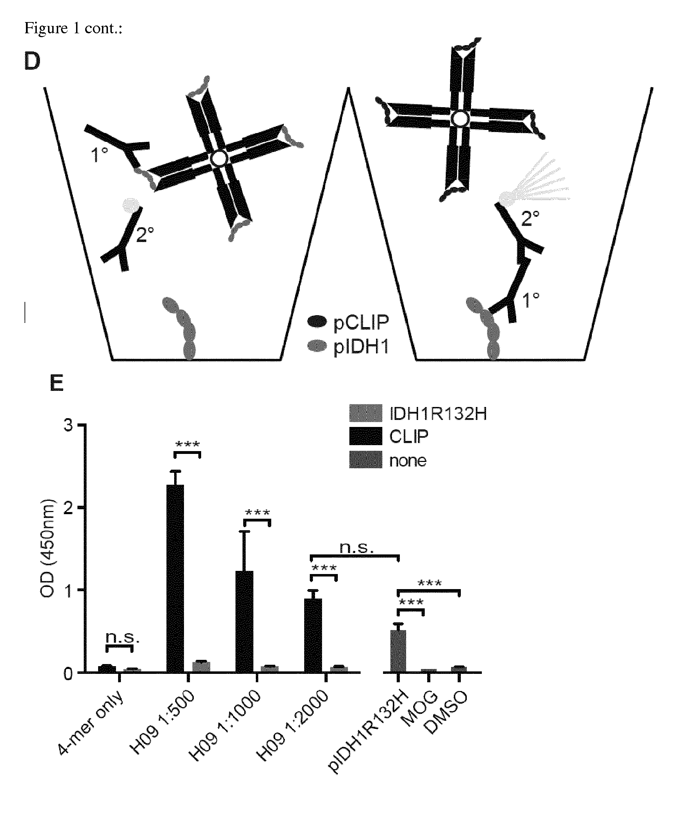

- **Figure 1:**: Binding of soluble and MHC class II-bound IDH1R132H 15-mer and 20-mer peptides by anti-IDH1R132H antibody (H09) in peptide-coated ELISA. (A) IDH1R132H IHC using H09 on glioma tissue p001 and p018; right panel, magnification of depicted area. (B) IDH1 15- and 20-mer peptide library encompassing aa 132. (C) IDH1 15- and 20-mer peptide coated ELISA. Black, IDH1 WT peptides; red, IDH1R132H peptides; MOG, negative control; DMSO, vehicle control. (D,E) MHC class II-bound IDH1R132H p122-136 peptide coated ELISA. H09 (1°) was pre-incubated with specific (red, IDH1R132H-HLA-DR1) and control (black, CLIP-HLA-DR1) tetramer (4-mer) and subsequently subjected to p122-136 IDH1R132H (pIDH1) coated ELISA; blue, no tetramer; MOG, control; DMSO, vehicle.

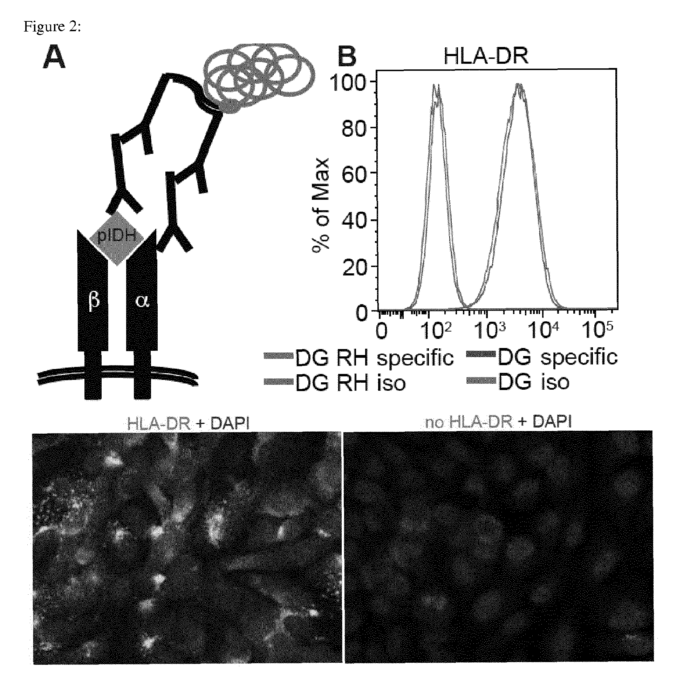

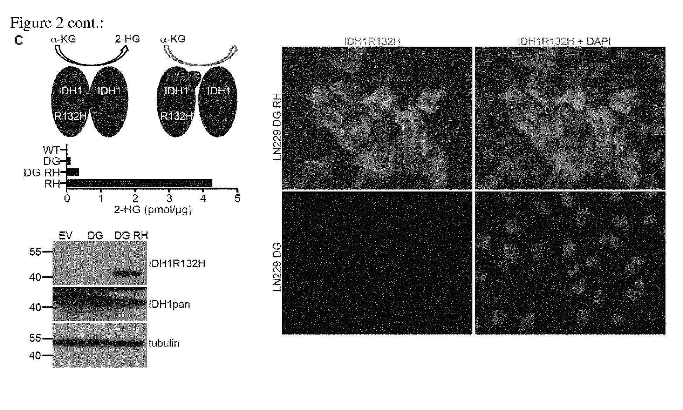

- **Figure 2:**: A tool for analysis of MHC class II-peptide interaction by PLA: HLA-DR expression and overexpression of mutated and wildtype IDH1 in glioma cell line LN229. (A) PLA scheme using anti-HLA-DRA and IDH1R132H-specific primary antibodies. α, β, HLA-DR chains; pIDH, IDH7 epitopic peptide; red, rolling circle amplification. (B) Immunofluorescent staining (LN229 IDH1 D252G R132H) and flow cytometry (LN229 IDH1 D252G R132H, DG RH; LN229 IDH1 D252G, DG) of glioma cell line LN229 endogenously expressing HLA-DR. (C) Left upper panel, scheme for enzymatic activity of IDH1 mutants and 2-HG measurement in IDH1 D252G R132H (DG RH), IDH1 D252G (DG), IDH1 R132H (RH), and IDH1 VVT (VVT) LN229 by enzymatic assay; lower left panel, western blot; right panel, immunofluorescent staining of LN229 overexpressing IDH1 D252G (DG) or IDH1 D252G R132H (DG RH). EV, empty vector.

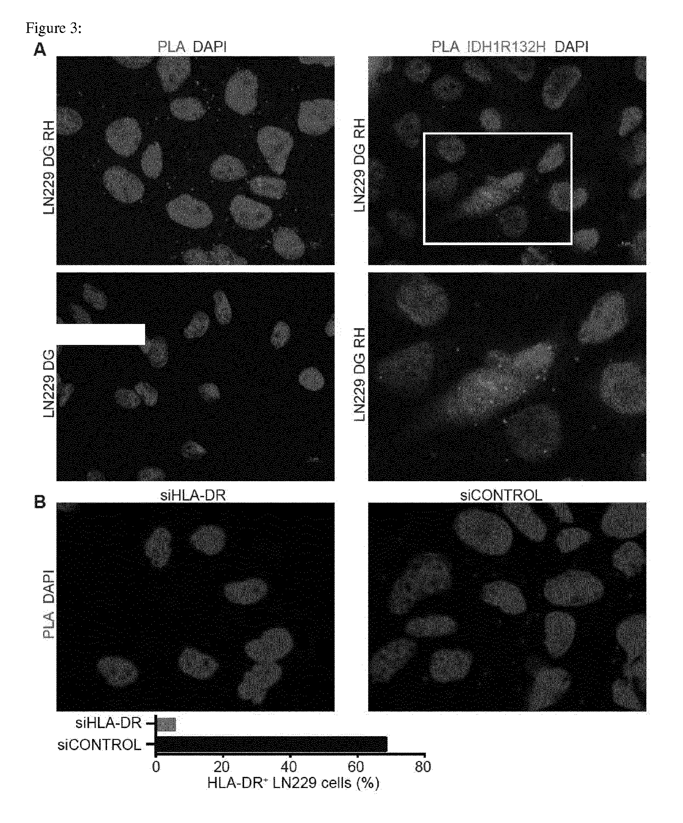

- **Figure 3:**: Specific co-localization of IDH1R132H peptide and MHC class II in IDH1 D252G R132H-overexpressing glioma cell line LN229 in vitro. (A) Left panel, IDH1 R132H-HLA-DR PLA on LN229 IDH1 D252G R132H (DG RH) and LN229 IDH1 D252G (DG), respectively. Upper right panel, IDH1 R132H-HLA-DR PLA with IDH1 R132H co-staining (green). Lower right panel, magnification of depicted area. (B) IDH1 R132H-HLA-DR PLA using HLA-DRA-specific siRNA or siRNA control pool on LN229 IDH1 R132H D252G. Quantification of HLA-DRA knockdown in LN229 IDH1 R132H D252G by flow cytometry. Red, PLA signal; blue, DAPI.

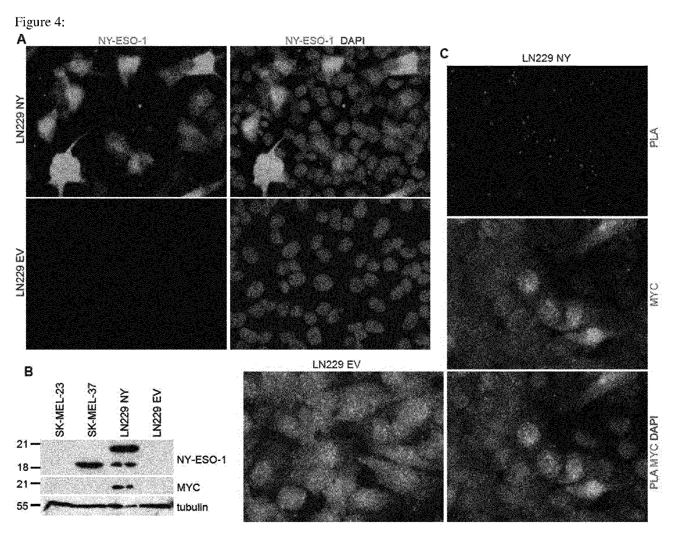

- **Figure 4:**: Specific co-localization ofNY-ESO-1 peptide and MHC class II in NY-ESO-1 overexpressing glioma cell line LN229 in vitro. (A) Immunofluorescent staining of LN229 stably overexpressing NY-ESO-1. (B) Western blot detecting endogenous NY-ESO-1 in SK-Mel-23 and SK-Mel-37 and myc-tagged (MYC) NY-ESO-1 overexpressed in LN229. Tubulin, loading control; LN229 EV, negative control. (C) NY-ESO-1-HLA-DR PLA on LN229 NY-ESO-1 and empty vector co-stained with anti-myc. NY, NY-ESO-1; EV, empty vector.

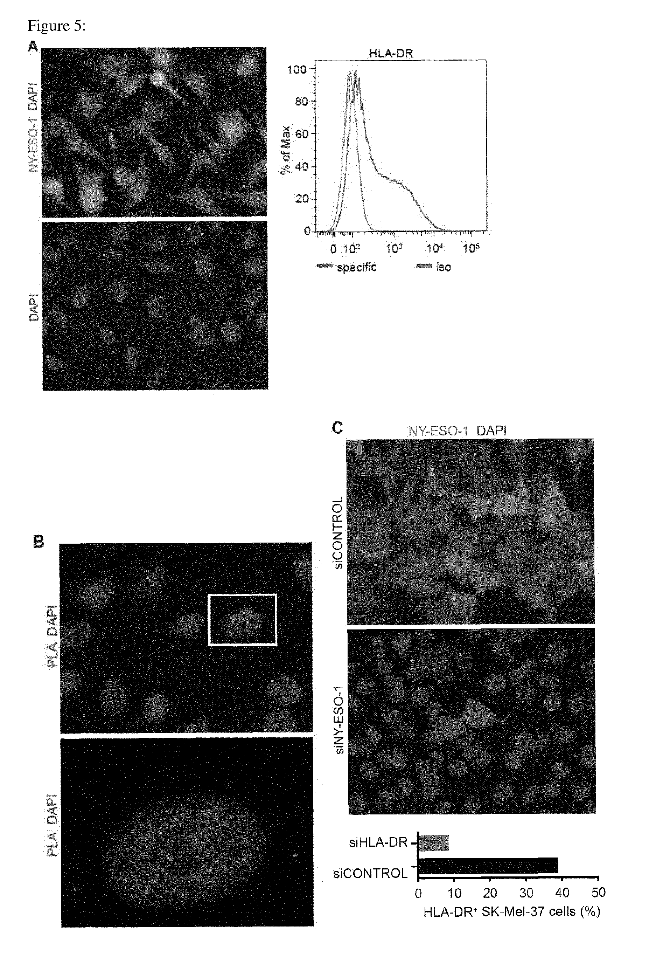

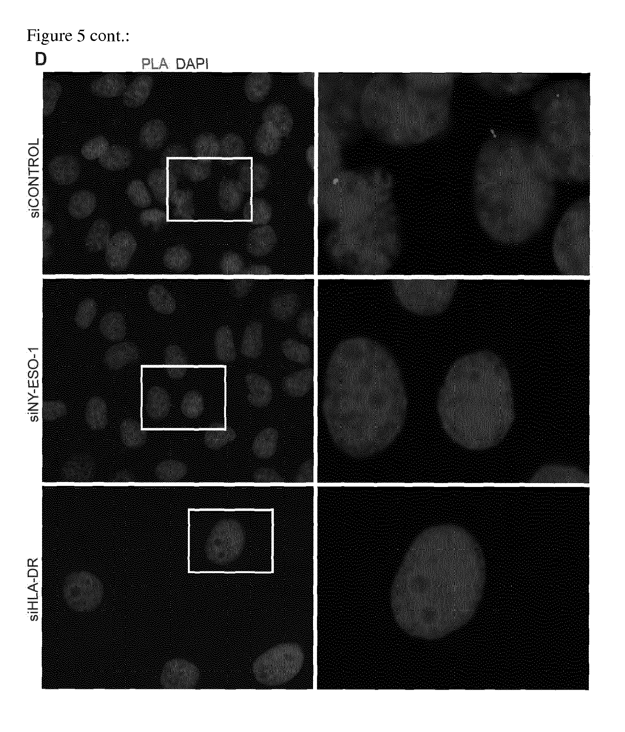

- **Figure 5:**: Specific co-localization of NY-ESO-1 peptide and MHC class II in melanoma cell line SK-Mel-37 endogenously expressing NY-ESO-1 and HLA-DR in vitro. (A) Upper left panel, immunofluorescent staining of SK-Mel-37 detecting endogenously expressed NY-ESO-1; lower left panel, negative control without primary antibody; right panel, flow cytometry of endogenous HLA-DR expression in SK-Mel-37. Specific, HLA-DR-specific antibody (red); iso, isotype (green). (B) NY-ESO-1-HLA-DR PLA on SK-Mel-37; below magnification of depicted area. Red, PLA signal, blue, DAPI. (C) SiRNA knockdown of HLA-DRA or NY-ESO-1 in SK-Mel-37. Upper left panel, immunofluorescent staining of SK-Mel-37 treated with siRNA control pool (siCONTROL) and NY-ESO-1 siRNA (siNY-ESO-1); lower panel, quantification of HLA-DRA knockdown in SK-Mel-37 by flow cytometry. (D) PLA on siCONTROL-, siNY-ESO-1-, and siHLA-DRA-treated SK-Mel-37. Red, PLA signal. Right panel, magnification of depicted areas.

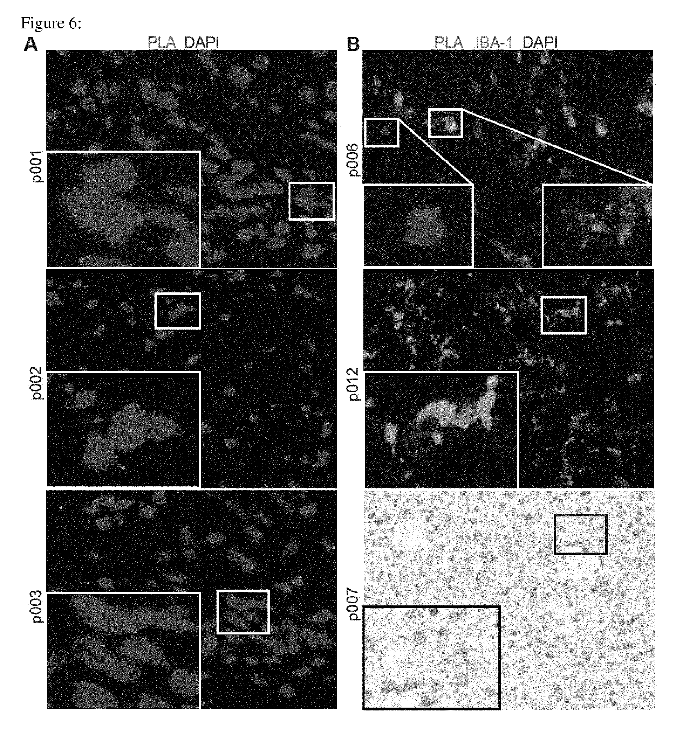

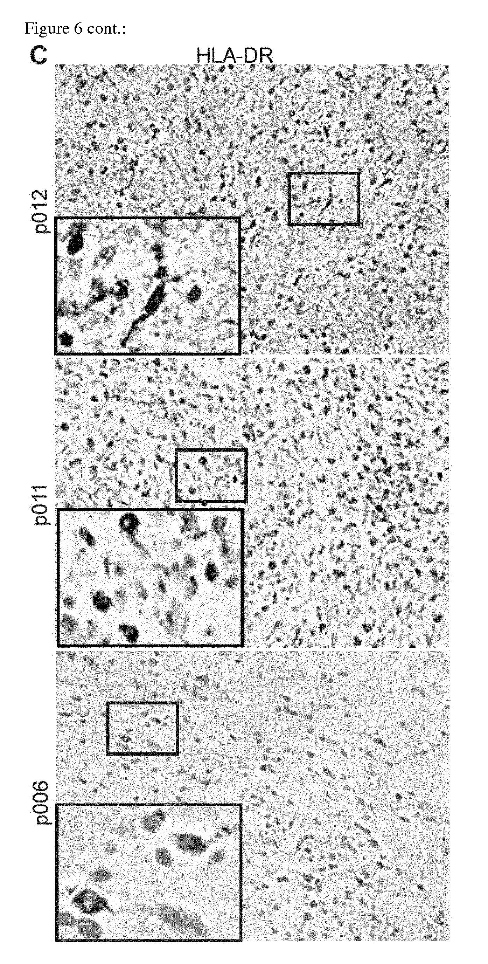

- **Figure 6:**: Specific co-localization of IDH1R132H and MHC class II in glioma tissue. (A) PLA on HLA-DR+ glioma tissue p001 (IDH1R132H), p002 (IDH1R132H), and p003 (IDH1 WT). Red, PLA signal. (B) PLA on HLA-DR+ glioma tissue p006 (IDH1R132H), and p012 (IDH1 WT) co-stained with IBA-1. Green, IBA-1; red, PLA signal. (C) Representative HLA-DR IHC of glioma tissue p006, p007, p011, and p012. Inlays, magnification of depicted areas.

### EXAMPLES

### Materials and Methods

### Peptides

Human IDHlwt and IDH1R132H amino acid sequences IDH1 p118-146 PRLVSGWVKPIIIGRHAYGDQYRATDFVV (SEQ ID NO: 1) and PRLVSGWVKPIIIGHHAYGDQYRATDFVV(SEQ ID NO: 2), respectively, cover the amino acid exchange from Arg to His at position 132, including all possible 15-mer IDH1 peptides containing position 132, and are identical to mouse sequence except for position 122 (Thr in mouse). Peptide libraries for ELISA and in vitro stimulation of IDHlwt and IDH1R132H of 10 and 20-mers contained the following peptides:
IDH1wt p118-132: PRLVSGWVKPIIIGR (SEQ ID NO: 3);
IDHlwt p120-134: LVSGWVKPIIIGRHA (SEQ ID NO: 4);
IDHlwt p122-136: SGWVKPIIIGRHAYG (SEQ ID NO: 5);
IDH1wt p124-138: WVKPIIIGRHAYGDQ (SEQ ID NO: 6);
IDH1wt p126-140: KPIIIGRHAYGDQYR (SEQ ID NO: 7);
IDHlwt p128-142: IIIGRHAYGDQYRAT (SEQ ID NO: 8);
IDHlwt p130-144: IGRHAYGDQYRATDF (SEQ ID NO: 9);
IDHlwt p132-146: RHAYGDQYRATDFVV (SEQ ID NO: 10);
IDH1R132H p118-132: PRLVSGWVKPIIIGH (SEQ ID NO: 11);
IDH1R132H p120-134: LVSGWVKPIIIGHHA (SEQ ID NO: 12);
IDH1R132H p122-136: SGWVKPIIIGHHAYG (SEQ ID NO: 13);
IDH1R132H p124-138: WVKPIIIGHHAYGDQ (SEQ ID NO: 14);
IDH1R132H p126-140: KPIIIGHHAYGDQYR (SEQ ID NO: 15);
IDH1R132H p128-142: IIIGHHAYGDQYRAT (SEQ ID NO: 16);
IDH1R132H p130-144: IGHHAYGDQYRATDF (SEQ ID NO: 17);
IDH1R132H p132-146: HHAYGDQYRATDFVV (SEQ ID NO: 18);

20-mers:
IDH1R132H p123-142 GWVKPIIIGHHAYGDQYRAT (SEQ ID NO: 19) and
IDHlwt p123-142 GWVKPIIIGRHAYGDQYRAT (SEQ ID NO: 20).

Negative control peptide for ELISA and in vitro stimulation was mouse myelin oligodendrocyte glycoprotein (MOG) p35-55 MEVGWYRSPFSRVVHLYRNGK (SEQ ID NO: 21) MOG peptide was synthesised by Genscript, IDH1 (wt, IDH1R132H) 15- and 20-mers were synthesised by Bachem Distribution Services GmbH. Peptides were diluted in PBS 10% DMSO at 2.5 mg/ml and stored at -80°C.

### In silico MHC class II peptide binding prediction

Human IDH1R132H 29-mer peptide PRLVSGWVKPIIIGHHAYGDQYRATDFVV includes all possible processed IDH1R132H 15-mers including the point mutation at codon 132 which leads to the amino acid change from arginin (R) to histidin (H). Binding of 15-mer IDH1R132H peptides to available HLA-DR types was predicted by the NetMHCII 2.2 algorithm.

### IDH1R132H and IDH1 wildtype peptide coated ELISA

ELISA plates (Costar) were coated with IDH1R132H and IDHlwt p118-132, p120-134, p122-136, p124-138, p126-140, p128-142, p130-144, p132-146, p123-142 (10 µg per well in PBS), washed with PBS 0.05 % Tween 20, and blocked with 3 % FBS in PBS 0.05 % Tween 20. As negative controls, MOG p35-55 was used at equal concentrations and peptide diluent PBS 10% DMSO was used at equal volume. As primary antibody monoclonal mouse anti-IDH1R132H (1:1000, H09, Dianova) was used. HRP-conjugated secondary antibody was sheep anti-mouse IgG (1:5000, Amersham). HLA-DRB1*01:01 MHC class II tetramer bound to IDH1R132H p123-142 and HLA-DRB1*01:01 MHC class II tetramer control tetramer bound to CLIP were kindly provided by NIH tetramer core facility and used 1:200 during pre-incubation with H09 for competitive ELISA. Substrate was TMB (ebioscience) and reaction was stopped with 1M H2SO4. OD at 450 nm was measured with an ELISA reader (Thermo Fisher).

### Glioma tissue

Assays were performed with glioma tissue from patients diagnosed at the Department of Neuropathology at Heidelberg University. IDH1 mutation status was routinely diagnosed by IHC. Tissues were obtained from the archives of the Department of Neuropathology, University Hospital Heidelberg, according to the regulations of the Tissue Bank of the National Center for Tumour Diseases, University Hospital Heidelberg, and used after approval of the local regulatory authorities.

### Cell lines and modification of gene expression

Glioma cell line LN229 endogenously expressing HLA-DR1 was lentivirally transduced with full-length cDNA of human IDH1R132H or IDHlwt (NCBI GenBank CR641695.1) in pLenti6.2/V5-DEST for stable expression. Virus was produced by transfection of HEK293T packaging cell line. Since IDH1R132H protein dimerizes and produces the oncometabolite R-2-HG which impacts proliferation and thereby impairs stable IDH1R132H expression, a second point mutation leading to the amino acid exchange D252G was introduced into IDH1R132H and wt-coding sequences by site-directed mutagenesis. This dimerization-deficient mutation results in an inert enzyme, thus to increased expression stability. Transduced cells were selected with 10 µg/ml blasticidin (Sigma-Aldrich) for stable overexpression. Stable overexpression of cancer testis antigen CTAG1A (NY-ESO-1) in LN229 was done by transfection with full length NY-ESO-1 cDNA (NCBI Genbank BC160040) provided by the DKFZ Genomics Facility, in the retroviral vector pMXs-IRES-BsdR (Cell Biolabs, Inc.) using FuGene HD transfection reagent (Promega). Cells were selected with 9 µg/ml blasticidin (Sigma-Aldrich) 72 h after transfection. Melanoma cell line SK-Mel-37 endogenously expressing NY-ESO-1 and HLA-DR1 and -DR3 was used for analysis of endogenous presentation of NY-ESO-1- derived peptides. For knockdown of HLA-DR in LN229 and HLA-DR and NY-ESO-1 in SK-Mel-37, ON-TARGET SMARTpool® siRNA (Dharmacon RNA Technologies,Lafayette, CO, USA) were used. The siRNA target sequences were as follows:
for CTAG1B (NY-ESO-1):
   CUGAAUGGAUGCUGCAGAU (SEQ ID NO: 22);
   CCGGCAACAUACUGACUAU (SEQ ID NO: 23);
   CGCCAUGCCUUUCGCGACA (SEQ ID NO: 24);
   GCUGGAGGAGGACGGCUUA (SEQ ID NO: 25);
for HLA-DRA:
   UGACAAAGCGCUCCAACUA (SEQ ID NO: 26);
   UGACCAAUCAGGCGAGUUU (SEQ ID NO: 27);
   GGAAUCAUGGGCUAUCAAA (SEQ ID NO: 28);
   CAACUGAGGACGUUUACGA (SEQ ID NO: 29);

As negative control ON-TARGETplus siCONTROL Non-targeting Pool (D-001810-10-05, Dharmacon) was used. The transfection was performed with lipofectamine RNAiMAX (Invitrogen) according to the manufacturer's protocol.

### Proximity ligation assay

Tumour cell lines were seeded on glass coverslips, grown until 70 - 90 % confluent and fixed and permeabilized with Cytofixx Pump Spray (Cell Path) for 30 min at -20°C and subsequent 4% PFA in PBS for 30 min at room temperature. Glioma tissue was deparaffinized with HistoClearTMII (National Diagnostics) and rehydrated. Antigen retrieval was performed using Cell Conditioning Solution CC1 (Ventana Medical Systems, Inc.) for 30 min. PLA was performed using Detection Reagents Red, PLA Probe anti-mouse PLUS, PLA Probe anti-rabbit MINUS and Wash Buffers Flourescence (all Duolink, Olink Bioscience) according to manufacturer's instructions. Briefly, blocking was done with blocking solution for 30 min at 37°C and primary antibodies monoclonal mouse anti-human IDH1R132H (1:100, H09, Dianova) or mouse anti-human monoclonal NY-ESO-1 (1:50, E978, Sigma Aldrich) with monoclonal rabbit anti-human HLA-DR (1:50, EPR3692, Abcam) in antibody diluent were incubated over night at 4°C. PLA Probe anti-mouse PLUS and PLA Probe anti-rabbit MINUS were incubated for 1 h at 37°C. Ligation and amplification were performed using the Detection Reagents Red. Immunofluorescent (IF) co-staining was performed as described below after amplification of PLA signal. Vectashield HardSet Mounting Medium with DAPI (Vector laboratories) was used for mounting and nuclear staining.

### Immuno fluorescent staining

For IF staining cells were seeded on glass coverslips, grown until 70 - 90 % confluent and fixed and permeabilized as described above. For blocking and staining blocking solution (Duolink, Olink Bioscience) and antibody diluent (Duolink, Olink Bioscience) were used as for PLA, respectively. As primary antibodies anti-IDH1R132H was used as described above. Additional IF stainings were performed using monoclonal rabbit anti-myc-tag (1:200, 71D10, Cell Signalling) and polyclonal rabbit anti-human IBA-1 (1:100, Wako) and secondary antibodies used were donkey anti-mouse AlexaFluor® 488 and goat anti-rabbit AlexaFluor 546® (all 1:300, Molecular Probes, Invitrogen). DAPI staining and mounting were performed as described above. For PLA co-staining secondary antibody anti-mouse AlexaFluor® 488 for IDH1R132H or myc-tag detection and anti-rabbit AlexaFluor® 488 (all 1:300, Molecular Probes, Invitrogen) for IBA-1 detection were used.

### Immunohistochemistry

Glioma tissue was deparaffinized with HistoClearTMII (National Diagnostics) and rehydrated. Antigen retrieval was performed using Cell Conditioning Solution CC1 (Ventana Medical Systems, Inc.) for 30 min. Endogenous peroxidase was blocked with 3 % hydrogen peroxide in PBS. Blocking was performed with 5 % FBS for one hour. Primary antibodies (monoclonal mouse anti-human IDH1R132H (1:100, H09, Dianova) and monoclonal rabbit anti-human HLA-DR (1:50, EPR3692, Abcam) were incubated over night at 4 °C. Colour reaction was performed using Liquid DAB+ Substrate Chromogen System (DAKO). Counterstaining was performed using hemalum (Carl Roth GmbH + Co. KG).

### Western Blot

Total protein was isolated by cell lysis with ice cold TRIS-HCl, 50 mM, pH 8,0 (Carl Roth) containing 150 mM NaCl (J.T. Baker, Deventer, Netherlands), 1 % Nondiet P-40 (Genaxxon Bioscience, Ulm, Germany), 10 mM EDTA (GerbuBiotechnik, Gaiberg, Germany), 200 mM dithiothreitol (Carl Roth), 100 µM PMSF and complete EDTA-free (1:50, Roche, Mannheim Germany) for 20 min and centrifuged to pellet debris. Protein concentrations were measured via the Bio-Rad protein assay (Bio-Rad, Hercules, CA, USA) at 595 nm and 30 µg of protein diluted in Laemmli sample buffer were denatured at 95 °C for 5 min and electrophoretically separated on 12 % acrylamide-polyacrylamide SDS-containing gels. Proteins were blotted onto nitrocellulose membranes by wet blot at 1.5 mA/cm2 for 1 h. After blocking with 5 % milk powder in 0.5 M TBS, pH 7.4, 1.5 M NaCl, 0.05 % Tween 20, membranes were incubated consecutively with primary monoclonal mouse anti-IDH1R132H (1:500, H09, Dianova), monoclonal rat anti-panIDH1 (1:500, WO9, Dianova) for detection of wt and R132H IDH1, monoclonal rabbit anti-myc tag (71D10, 1:1000, Cell Signalling), anti-NY-ESO-1 (1:500, Sigma Aldrich) overnight at 4 °C, and mouse anti-α-tubulin (1:5000, Sigma-Aldrich) as loading control for 1h at room temperature. Staining with secondary HRP-conjugated anti-rat (1:(1000xF), Dako) or anti-mouse (1:5000, GE Healthcare, Buckinghamshire, UK) antibodies was performed at room temperature for 1 h and was followed by chemiluminescent development using ECL or ECL prime (both Amersham).

### Flow cytometry

Cells were harvested, washed once in PBS, 3% FBS, 2 mM EDTA, and blocking was done with human serum. Surface HLA-DR was stained using eFluor-450®-conjugated mouse anti-HLA-DR antibody (1:100, L243, ebioscience). Cells were acquired on a FACS Canto II (Beckton Dickinson) and analysed using FlowJo software.

### HLA-typing

Genomic DNA was isolated from patient blood or tumor samples using the FFPE LEV DNA Purification KIT AS1130, from cell lines using the QIAamp DNA Mini Kit (Qiagen). PCR-based typing was performed using HLA-A and HLA-DR type-specific primer pairs lyophilized in a 96 well plate (HLA-A* CTS-PCR-SSP Minitray Kit and HLA-DRB1* CTS-PCR-SSP Minitray Kit) and Mastermix 5,0% for HLA-DRB1* and Mastermix 7,5% for HLA-A* (all from Department of Transplantation Immunology, University Clinic Heidelberg) with Taq-polymerase (Fermentas) PCR was performed according to manufacturer's instructions. PCR products were separated on a 1.5 % agarose gel containing GelRed® (1:10000, Genaxxon bioscience). Analysis was done according to manufacturer's instruction.

### 2-HG measurement

2-HG production in cells was analyzed as described previously [33]. As a control for 2-HG production enzymatically competent retrovirally transduced LN229 IDH1 R132H were used. Vectors were generated as described previously (Schumacher Bunse nature 2014 Referenz).

### Image analysis

IF images were taken on LEICA DM IRB microscope, using 63x objective for PLA, detecting PLA signal with N2.1 filter, signal of immunofluorescent co-staining with GFP filter, using 40x objective for immunofluorescent images. Immunohistochemical images were taken on Zeiss Axioplan microscope. Images were linearly optimised with Adobe Photoshop CS3®.

### Statistical analysis

Data are expressed as mean + s.e.m. and analysis of significance (Fig. 1C, E) was performed using the one-way ANOVA, Tukey corrected (Prism 6.0). PLA positivity was set to 20 PLA signals per high power field and analysis of significance was performed using Fisher's exact test, (Table 2, R version 2.15.2.). P values < 0.05 were considered significant.

### Example 1: MHC class II-restricted immunogenicity of IDH1R132H

The IDH1R132H mutation is expressed in about 80% of gliomas, defining a distinct glioma subtype [15-17]. The high incidence of this point mutation led to the development of a mutation-specific monoclonal antibody (H09) [18, 19], which finds routine application for histological diagnostics. This mouse antibody has been generated by immunization with synthetic peptide IDH1R132H p125-137 CKPIIIGHHAYGD (SEQ ID NO: 30) coupled to keyhole limpet hemocyanin and allows specific staining of diffuse infiltrating single tumor cells in gliomas (Fig 1A). To assess putative immunogenicity of IDH1R132H in a human MHC class II context, epitopes were identified in silico by MHC peptide binding predictions. Human IDH1R132H 29-mer peptide (118-146) PRLVSGWVKPIIIGHHAYGDQYRATDFVV includes all possible processed IDH1R132H 15-mers covering the point mutation at codon 132. *In silico* peptide binding algorithms predicted IDH1R132H 15-mer binding to human MHC class II in an HLA-DR type-dependent manner (Table 1).

**Table 1: IDH1R132H 15-mer peptides bind to human MHC class II in silico. NetMHCII algorithm was used to predict binding of IDH1R132H 15-mer peptides to available HLA-DR types. Peptides with IC50 below 500 nM are defined as weak binders, those with IC50 below 50 nM are defined as strong binders. Only 15-mers predicted to bind are shown.**

| allel | 15-mers | position | IC50 (nM) | binding level |
|---|---|---|---|---|
| HLA-DRB1*0101 | PRLVSGWVKPIIIGH | 118-132 | 39.1 | s |
| | RLVSGWVKPIIIGHH | 119-133 | 60.4 | w |
| HLA-DRB1*0701 | PRLVSGWVKPIIIGH | 118-132 | 331.5 | w |
| | RLVSGWVKPIIIGHH | 119-133 | 392.6 | w |
| HLA-DRB1*0802 | SGWVKPIIIGHHAYG | 122-136 | 192.5 | w |
| | VSGWVKPIIIGHHAY | 121-135 | 217.6 | w |
| HLA-DRB1*1101 | VKPIIIGHHAYGDQY | 125-139 | 222.7 | w |
| | WVKPIIIGHHAYGDQ | 124-138 | 250.7 | w |
| HLA-DRB1*1501 | VKPIIIGHHAYGDQY | 125-139 | 37.2 | s |
| | KPIIIGHHAYGDQYR | 126-140 | 38.6 | s |
| HLA-DRB4*0101 | KPIIIGHHAYGDQYR | 126-140 | 145.3 | w |
| | VKPIIIGHHAYGDQY | 125-139 | 146.4 | w |
| HLA-DRB5*0101 | SGWVKPIIIGHHAYG | 122-136 | 309.9 | w |
| | VSGWVKPIIIGHHAY | 121-135 | 311.0 | w |

The inventors then sought to address IDH1R132H epitope processing and presentation *in vitro* using PLA. PLA has been developed for protein-protein interaction analysis in unmodified cells and tissues by applying specific antibodies for proteins of interest coupled to PCR probes. Rolling circle PCR amplification then allows for the fluorescent visualization of co-localization of native proteins in situ. Thus, in principle, this technique is applicable for the analysis of natural peptide processing, as it does not require structural modifications of the system by exogenous expression or introduction of fluorescent labels. In order to employ the PLA for epitope presentation on MHC, a specific antibody detecting the epitope of interest is required. A peptide library was generated encompassing the IDH1R132H region (Fig. 1B). Peptide-coated ELISA assays demonstrated that the anti-IDH1R132H antibody binds to the IDH1-mutated 15-mers p122-136, p124-138 and p126-140 and the 20-mer p123-p142, whereas no binding was seen for any of the IDHlwt peptides nor IDH1R132H peptides with a peripheral position of the amino acid exchange (Fig. 1C). Specific binding of the HLA-DR-bound IDH1R132H 20-mer p123-142 by the employed IDH1R132H-specific antibody is a prerequisite for analysis of peptide-MHC class II interaction. The inventors sought to address this question by an immune-competitive ELISA approach, employing p123-142 IDH1R132H-loaded class II (DR1) tetramers (Fig. 1D). The inventors have previously employed this tetramer to identify IDH1R132H-specific CD4+ T cells (Schumacher, Bunse nature 2014). Pre-incubation of IDH1R132H-DR1-tetramer but not control (CLIP)-tetramer with IDH1R132H-specific antibody resulted in complete inhibition of specific antibody binding to ELISA plate-coated IDH1R132H peptide p122-136 (Fig. 1E). This result supports the hypothesis that an IDH1R132H-specific antibody recognizes an unmasked IDH1R132H-epitope in an MHC class II-bound setting.

### Example 2: Establishment of an in vitro system to detect IDH1R132H-MHC class II co-localization.

As an *in vitro* system to evaluate the applicability and specificity of PLA for detection of IDH1R132H epitope presentation on HLA-DR (Fig. 2A), the inventors employed the human glioma cell line LN229, which endogenously and homozygously expresses HLA-DRB1*01 (Fig. 2B). LN229 cells, which are IDH1wt, were stably transduced with the double mutant IDH1D252G / R132H (Fig. 2C). The inventors introduced the point mutation at position 252 to abolish the neomorphic enzymatic activity of IDH1R132H thus to increase IDH1R132H expression which is negatively influenced by high amounts of R-2-hydroxyglutarate (R-2-HG) produced by IDH1R132H (Fig. 2C).

### Example 3: IDH1R132H-MHC class II co-localization can be detected in vitro.

In IDH1D252G / R132H transduced cells, but not cells expressing IDH1D252G, / WT an epitope-specific proximity of MHC class II HLA-DR and IDH1R132H peptide was detected using PLA (Fig. 3A). Immunofluorescent counter-staining with the IDH1R132H-specific antibody revealed a correlation of IDH1R132H-expression levels and the PLA signal. An HLA-DR-specific knockdown abolished the PLA-signal in IDH1D252G / R132H-overexpressing LN229 confirming PLA-specificity for HLA-DR (Fig. 3B). These results show that PLA is suitable to specifically detect co-localization of IDH1R132H epitopes and MHC class II HLA-DR and suggest that the HLA-DR-positive LN229 glioma cells are able to present these epitopes on HLA-DR.

### Example 4: PLA also demonstrates co-localization of an endogenously expressed tumor-associated antigen (TAA) in vitro.

To confirm the applicability of PLA as a tool to detect epitope presentation on HLA-DR, the inventors next aimed to extend this finding to an established TAA of known functional relevance. The cancer testis antigen NY-ESO-1 (CTAG1B) represents a suitable antigen, because it has been shown to be a potent immunogenic target in various studies, inducing not only specific CD8+ T cell-mediated, but also CD4+ T cell-mediated responses and containing several MHC class II HLA-DR-binding epitopes e.g. p119-143, p121-138, and p123-137 for HLA-DR1 [20, 21]. Therefore, the inventors overexpressed myc-tagged NY-ESO-1 in LN229 (Fig. 4A, B) and performed PLA, showing co-localization of NY-ESO-1 and endogenous HLA-DR1 using a specific antibody generated against full length human NY-ESO-1 (Fig. 4C). Signal intensity correlated with expression levels as shown by counter-staining for myc. To exclude that the detected interaction between an epitope derived from the TAA NY-ESO-1 and HLA-DR is a result of the forced overexpression of the antigen, the inventors performed in vitro PLA on endogenously antigen-expressing tumor cells. The melanoma cell line SK-Mel-37 endogenously expresses NY-ESO-1 and HLA-DRB1*01 and HLA-DRB1*03 (Fig. 4B, 5A). PLA demonstrated co-localization of HLA-DR and endogenous NY-ESO-1 (Fig. 5B). The signal was abolished by siRNA-mediated knockdown of either NY-ESO-1 or HLA-DR (Fig. 5C, D), confirming the capability of PLA to detect co-localization of endogenous epitopes presented on MHC class II and supporting its applicability as a tool to detect antigen-presentation *in vitro*.

### Example 5: IDH1R132H-MHC class II PLA demonstrates co-localization in situ.

The inventors next assessed the applicability of PLA to detect epitope presentation in tumor tissue in situ. To this aim, PLA was performed on paraffin-embedded glioma tissue. Fig. 6A demonstrates that IDH1R132H-MHC class II co-localization was found specifically in IDH1R132H+ and HLA-DR+ (p001, p002) but not in an IDHlwt HLA-DR+ (p003) glioma tissue. Subsequently, the inventors sought to identify the IDH1R132H epitope-presenting cell type in the tumor tissue. To this end, glioma tissue was subjected to PLA and counter-stained for the microglia marker Iba-1 (Fig. 6B). Co-staining with Iba-1 showed that PLA signals are not restricted to microglia, i.e. professional APC, suggesting that class II expressing glioma cells themselves may present IDH1R132H. The analysis of a cohort of 18 patients (9 patients with IDHlwt gliomas, 9 patients with IDH1R132H+ gliomas, 15 of which HLA-DR+) revealed a positivity in the IDH1R132H-MHC class II PLA system in 5 out of 9 IDH1R132H+, but in none of 9 tested IDHlwt gliomas (p = 0.029 Fisher's exact test). Moreover, PLA signal was only detected in tissue that was also stained positive for HLA-DR by immunohistochemistry (IHC) (Figure 6C, Table 2). These results confirm the specificity of PLA not only in vitro, but also *in situ.*

With the increased interest in targeting true tumor antigens, which typically are mutated antigens, by active immunotherapy [22-24] there is an increasing requirement to evaluate whether these antigens are indeed presented in the tumor tissue. In contrast to TAA, mutated antigens have not undergone central tolerance, however, they are often minor antigens presented on MHC class II rather than MHC class I [25]. While the latter has been considered a disadvantage for a long time there is an increasing evidence that an antigen-specific CD4+ T cell response is capable of executing an effective antitumor immunity and not just provide help for CD8+ T cells [26, 27]. Potential mechanisms include direct cytotoxicity by antigen-specific CD4+ T cells towards tumor cells presenting the antigen on MHC class II and activation of innate immune cells by antigen-specific CD4+ T cells stimulated through intratumoral professional APC presenting tumor antigens. The inventors have previously demonstrated that IDH1, which is frequently mutated in gliomas and other types of tumors, represents a novel tumor neo-antigen. The IDH1R132H mutation, which represents the most common mutation in IDH1, is capable of inducing a mutation-specific class II-restricted CD4+ T cell response in patients with IDH1R132H-mutated gliomas and in MHC-humanized A2.DR1 mice after vaccination (Schumacher Bunse Nature 2014). Two lines of evidence in our previous study suggest that IDH1R32H is endogenously processed and presented on MHC class II: (i) In patients with IDH1R132H+ gliomas but not IDHlwt gliomas, mutation-specific CD4+ T cells can be detected after ex vivo stimulation with IDH1R132H (p123-142) in an MHC class II-restricted manner. (ii) Mutation-specific CD4+ T cells can be detected after vaccination of MHC-humanized mice with irradiated syngeneic sarcomas expressing human IDH1R132H. Here the inventors present a novel approach to detect presentation of antigenic epitopes in situ applying PLA, a combined immunofluorescence and PCR method.

**Table 2: Mutational and genetic analysis and clinical information of glioma patients. F, female; m, male; A, astrocytoma, GBM, glioblastoma (WHO °IV); IHC, immunohistochemistry; semi-quantitative analysis of HLA-DR expression: -, negative; +, low; ++, moderate; +++, strong; ++++, very strong; *, mainly microglial HLA-DR expression; n.d., not determined.**

| patients | gender | age | diagnosis | IDH1 status (IHC) | PLA signal | HLA-type | HLA-DR expression |
|---|---|---|---|---|---|---|---|
| p001 | f | 25 | A°II | IDH1R132H | + | DRB1*07,10:01 DRB3* | + |
| p002 | m | 50 | GBM | IDH1R132H | + | DRB1*10:01,15 DRB5* | + |
| p003 | m | 71 | GBM | IDH1wt | - | n.d. | + |
| p004 | m | 41 | A°III | IDH1R132H | - | DRB1*03,07 DRB4* | +++ |
| p005 | f | 52 | A°III | IDH1R132H | - | DRB1*07,07 | +* |
| p006 | m | 35 | A°III | IDH1R132H | + | DRB1*03,11, DRB3* | + |
| p007 | f | 55 | A°III | IDH1R132H | - | DRB1*11,13 DRB3* | - |
| p008 | m | 42 | A°III | IDH1wt | - | n.d. | ++* |
| p009 | f | 58 | A°III | IDH1wt | - | n.d. | + |
| p010 | m | 42 | A°III | IDH1wt | - | n.d. | ++++ |
| p011 | m | 48 | A°III | IDH1wt | - | n.d. | ++ |
| p012 | f | 88 | A°III | IDH1wt | - | n.d. | +* |
| p013 | f | 60 | A°III | IDH1wt | - | n.d. | - |
| p014 | m | 55 | A°III | IDH1wt | - | n.d. | + |
| p015 | m | 45 | A°III | IDH1wt | - | n.d. | - |
| p016 | m | 40 | A°III | IDH1R132H | + | n.d. | + |
| p017 | m | 42 | A°III | IDH1R132H | + | DRB1*01,15 DRB5* | + |
| p018 | f | 71 | A°III | IDH1R132H | - | n.d. | +++ |

While this approach offers the advantage of detecting presentation *in situ* on paraffin-embedded tissue slides it is restricted in its use by the requirements for the antibody targeting the antigen: (i) The antibody targeting the antigen must recognize the presented epitope bound to MHC (class II) in a native state and in situ. (ii) In case of mutated antigens, the antibody needs to be mutation-specific. For IDH1R132H both requirements are fulfilled. The mutation-specificity has been established in numerous studies [18, 19, 28]. In fact, IDH1R132H IHC is now implemented in routine diagnostics. In this study, the inventors demonstrate that the antibody recognizes the same epitope, which is presented on MHC class II, which is part of IDH1R132H p123-142 and the shorter peptides p122-136, p124-138 and p126-140 (Fig. 1B). Importantly, binding of the anti-IDH1R132H antibody to these epitopes can be blocked by IDH1R132H class II tetramers, further supporting the evidence that H09 recognizes the IDH1R132H epitope bound to MHC class II in a native state (Fig. 1E).

The inventors further extend this observation to a classic tumor-associated antigen, NY-ESO-1. PLA using an NY-ESO-1 specific and a HLA-DR-specific antibody detected co-localization of an NY-ESO-1 epitope with HLA-DR in SK-Mel-37 endogenously expressing NY-ESO-1 and HLA-DR1 and HLA-DR3 (Fig. 5). While there have been several class II epitopes characterized for NY-ESO-1, the epitope recognized by the NY-ESO-1 antibody is unknown to us. The observation that this antibody recognizes an epitope, which is also presented on MHC class II, is likely to be not just a coincidence but due to the fact that class II-restricted CD4 epitopes and B cell epitopes sometimes overlap [29]. In fact, screening of antibodies using PLA on tumor tissue may be a useful tool to map relevant epitopes presented in the tumor tissue.

The inventors also present evidence that IDH1R132H is presented in human IDH1R132H-mutated gliomas. While larger prospective series are required to determine the biological relevance of PLA-positivity, it is conceivable that this method may find application in preselecting patients for a targeted immunotherapy. With respect to deciphering the cellular context of antigen-presentation in the tumor tissue, there is room for improvement with respect to multi-labelling. The attempts to do so in this study suggest that IDH1R132H is not only presented by tumor cells themselves, which can be MHC class II positive (Fig. 6,[30, 31]) but also presented by microglial cells.

In summary, the inventors present a novel method to detect presentation of antigens in situ which may find application in but is not restricted to the identification of relevant tumor (associated) antigens.

### References:

1. Kantoff, P.W., et al., Sipuleucel-T immunotherapy for castration-resistant prostate cancer. N Engl J Med, 2010. 363(5): p. 411-22.
2. Mocellin, S., et al., Part I: Vaccines for solid tumours. Lancet Oncol, 2004. 5(11): p. 681-9.
3. Mocellin, S., et al., Part II: Vaccines for haematological malignant disorders. Lancet Oncol, 2004. 5(12): p. 727-37.
4. Prins, R.M., et al., Gene expression profile correlates with T-cell infiltration and relative survival in glioblastoma patients vaccinated with dendritic cell immunotherapy. Clin Cancer Res, 2011. 17(6): p. 1603-15.
5. Sampson, J.H., et al., An epidermal growth factor receptor variant III-targeted vaccine is safe and immunogenic in patients with glioblastoma multiforme. Mol Cancer Ther, 2009. 8(10): p. 2773-9.
6. Sliwkowski, M.X. and I. Mellman, Antibody therapeutics in cancer. Science, 2013. 341(6151): p. 1192-8.
7. Zhao, L.L., et al., Pathological significance of epidermal growth factor receptor expression and amplification in human gliomas. Histopathology, 2012. 61(4): p. 726-736.
8. Jager, E., et al., Monitoring CD8 T cell responses to NY-ESO-1: correlation of humoral and cellular immune responses. Proc Natl Acad Sci USA, 2000. 97(9): p. 4760-5.
9. Lundegaard, C., et al., NetMHC-3.0: accurate web accessible predictions of human, mouse and monkey MHC class I affinities for peptides of length 8-11. Nucleic Acids Res, 2008. 36(Web Server issue): p. W509-12.
10. Nielsen, M. and O. Lund, NN-align. An artificial neural network-based alignment algorithm for MHC class II peptide binding prediction. BMC Bioinformatics, 2009. 10: p. 296.
11. Rammensee, H., et al., SYFPEITHI: database for MHC ligands and peptide motifs. Immunogenetics, 1999. 50(3-4): p. 213-9.
12. Regner, M., et al., An improved method for the detection of peptide-induced upregulation of HLA-A2 molecules on TAP-deficient T2 cells. Exp Clin Immunogenet, 1996. 13(1): p. 30-5.
13. Dutoit, V., et al., Exploiting the glioblastoma peptidome to discover novel tumour-associated antigens for immunotherapy. Brain, 2012. 135(Pt 4): p. 1042-54.
14. Weiler, M., et al., mTOR target NDRG1 confers MGMT-dependent resistance to alkylating chemotherapy. Proc Natl Acad Sci USA, 2014. 111(1): p. 409-14.
15. Balss, J., et al., Analysis of the IDH1 codon 132 mutation in brain tumors. Acta Neuropathol, 2008. 116(6): p. 597-602.
16. Verhaak, R.G., et al., Integrated genomic analysis identifies clinically relevant subtypes of glioblastoma characterized by abnormalities in PDGFRA, IDH1, EGFR, and NF1. Cancer Cell, 2010. 17(1): p. 98-110.
17. Yan, H., et al., IDH1 and IDH2 mutations in gliomas. N Engl J Med, 2009. 360(8): p. 765-73.
18. Capper, D., et al., Characterization of R132H mutation-specific IDH1 antibody binding in brain tumors. Brain Pathol, 2010. 20(1): p. 245-54.
19. Capper, D., et al., Monoclonal antibody specific for IDH1 R132H mutation. Acta Neuropathol, 2009. 118(5): p. 599-601.
20. Ayyoub, M., et al., Assessment of vaccine-induced CD4 T cell responses to the 119-143 immunodominant region of the tumor-specific antigen NY-ESO-1 using DRB1*0101 tetramers. Clin Cancer Res, 2010. 16(18): p. 4607-15.
21. Zarour, H.M., et al., NY-ESO-1 119-143 is a promiscuous major histocompatibility complex class II T-helper epitope recognized by Th1- and Th2-type tumor-reactive CD4+ T cells. Cancer Res, 2002. 62(1): p. 213-8.
22. Choi, B.D., et al., EGFRvIII-targeted vaccination therapy of malignant glioma. Brain Pathol, 2009. 19(4): p. 713-23.
23. Sampson, J.H., et al., Immunologic escape after prolonged progression-free survival with epidermal growth factor receptor variant III peptide vaccination in patients with newly diagnosed glioblastoma. J Clin Oncol, 2010. 28(31): p. 4722-9.
24. Sensi, M. and A. Anichini, Unique tumor antigens: evidence for immune control of genome integrity and immunogenic targets for T cell-mediated patient-specific immunotherapy. Clin Cancer Res, 2006. 12(17): p. 5023-32.
25. Accolla, R.S. and G. Tosi, Optimal MHC-II-restricted tumor antigen presentation to CD4+ T helper cells: the key issue for development of anti-tumor vaccines. J Transl Med, 2012. 10: p. 154.
26. Hunder, N.N., et al., Treatment of metastatic melanoma with autologous CD4+ T cells against NY-ESO-1. N Engl J Med, 2008. 358(25): p. 2698-703.
27. Quezada, S.A., et al., Tumor-reactive CD4(+) T cells develop cytotoxic activity and eradicate large established melanoma after transfer into lymphopenic hosts. J Exp Med, 2010. 207(3): p. 637-50.
28. Sahm, F., et al., Addressing diffuse glioma as a systemic brain disease with single-cell analysis. Arch Neurol, 2012. 69(4): p. 523-6.
29. Lehtinen, M., et al., A T-helper cell epitope overlaps a major B-cell epitope in human papillomavirus type 18 E2 protein. APMIS, 1992. 100(11): p. 1022-6.
30. Rees, R.C. and S. Mian, Selective MHC expression in tumours modulates adaptive and innate antitumour responses. Cancer Immunol Immunother, 1999. 48(7): p. 374-81.
31. Takamura, Y., et al., Regulation of MHC class II expression in glioma cells by class II transactivator (CIITA). Glia, 2004. 45(4): p. 392-405.
32. Nielsen, M., C. Lundegaard, and O. Lund, Prediction of MHC class II binding affinity using SMM-align, a novel stabilization matrix alignment method. BMC Bioinformatics, 2007. 8: p. 238.
33. Balss, J., et al., Enzymatic assay for quantitative analysis of (D)-2-hydroxyglutarate. Acta Neuropathol, 2012. 124(6): p. 883-91.

## Claims

1. A method for detecting antigen presentation of an epitope by an antigen presentation molecule, comprising
(a) Providing a first binding agent and a second binding agent, wherein the first binding agent is capable of specifically binding the epitope, and the second binding agent is capable of specifically binding the antigen presentation molecule; wherein the first and the second binding agent are **characterized in that** spatial proximity of the first binding agent and the second binding agent induces a detectable signal,
(b) Providing an antigen presentation molecule, wherein the antigen presentation molecule is suspected of presenting the epitope,
(c) Providing the epitope,
(d) Bringing into contact the epitope, the antigen presentation molecule, the first binding agent and the second binding agent,
wherein the presence of the detectable signal is indicative for the antigen presentation of the epitope by the antigen presentation molecule.

2. The method according to claim 1, wherein the first binding agent comprises a first proximity probe and the second binding agent comprises a second proximity probe, wherein spatial proximity of the first proximity probe and the second proximity probe induces the detectable signal.

3. The method according to claim 1, further comprising providing a third binding agent capable of specifically binding to the first binding agent; providing a fourth binding agent capable of specifically binding to the second binding agent; and
wherein step (d) comprises bringing into contact the epitope, the antigen presentation molecule, the first binding agent, the second binding agent, the third binding agent, and the fourth binding agent.

4. The method according to claim 3, wherein the third binding agent comprises a first proximity probe and the fourth binding agent comprises a second proximity probe, wherein spatial proximity of the first proximity probe and second proximity probe induces the detectable signal.

5. The method according to any of claims 1 to 4, wherein the binding agents are monoclonal- or polyclonal antibodies, preferably monoclonal antibodies.

6. The method according to any of claims 1 to 5, wherein in step (b) the antigen presentation molecule is provided in a biological cell, preferably on the surface on a biological cell such as an antigen presenting cell selected from a dendritic cell, a B lymphocyte or a tumor cell.

7. The method according to any of claims 1 to 6, wherein the epitope is a disease associated antigen, preferably an epitope associated with an immunological disorder, such as an autoimmune disorder, or a tumor associated antigen (TAA), or an epitope derived from a TAA, preferably wherein the TAA is selected from the group of cancer mutated antigens, cancer germ line expressed antigens, cancer viral antigens or cancer overexpression antigens.

8. The method according to any of claims 1 to 7, wherein the epitope is derived from IDH1 and comprises the IDH1R132H mutation, most preferably a peptide comprising an amino acid sequence according to SEQ ID NO: 30 (peptide IDH1R132H p125-137), or wherein the epitope is derived fromNY-ESO-1.

9. The method according to any of claims 1 to 8, wherein the epitope is provided by providing a biological cell expressing the epitope or a precursor thereof; or alternatively ectopically expressing the epitope, or a precursor thereof, in a biological cell, preferably a cell which further comprises the antigen presentation molecule, most preferably an antigen presenting cell such as a dendritic cell, a B lymphocyte or a tumor cell.

10. A method for generating a personalized disease therapy plan for treating a subject suffering from a disease, the method comprising the steps of
(a) Providing a biological sample obtained from the subject,
(b) Detecting antigen presentation of at least one known epitope or antigen in the biological sample using the method according to any of claims 1 to 9, wherein the epitope and the antigen presentation molecule are provided in the biological sample, and
(c) Generating a therapy plan for treating the subject by selecting a vaccine composition comprising vaccine-molecules corresponding to the epitope or antigen as detected in (a).

11. A method for producing a personalized vaccine composition, the method comprising the steps of
(a) Providing a biological sample obtained from the subject,
(b) Detecting antigen presentation of at least one known epitope or antigen in the biological sample using the method according to any of claims 1 to 9, wherein the epitope and the antigen presentation molecule are provided in the biological sample, and
(c) Producing a personalized vaccine composition by admixing vaccine compounds into a composition which correspond to the epitopes/antigens as detected in (b).

12. The method according to claims 10 or 11, wherein the biological sample is a tissue sample, and the detecting antigen presentation is performed in the tissue sample *in-situ.*

13. The method according to any of claims 10 to 12, wherein the epitope is, or is derived of, a TAA, preferably a mutated tumor antigen.

14. A method for diagnosing, stratifying, monitoring or classifying a subject suffering from a disease, the method comprising the steps of:
(a) Providing a biological sample of the subject suffering from a disease to be diagnosed,
(b) Detecting antigen presentation of at least one known epitope or antigen in the biological sample, the epitope being characteristic for a candidate disease, using the method according to any of claims 1 to 9, wherein the epitope and the antigen presentation molecule are provided in the biological sample,
wherein a diagnosis is provided based on the presence or absence of the presentation of the epitope/antigen in said cellular sample or tissue sample

15. Use of a proximity ligation assay (PLA) in a method according to any of claims 1 to 9.

16. Use of a PLA kit in a method according to any of claims 1 to 9, the kit comprising at least the first binding agent and the second binding agent, optionally the kit further comprises the second binding agent and the fourth binding agent.

17. A diagnostic kit adapted to be for use in the performance of a method according to any of claims 1 to 14, the kit comprising at least the first binding agent, the second binding agent, optionally the third and fourth binding agent.
